(19) Europäisches Patentamt / European Patent Office / Office européen des brevets

(11) **EP 2 636 003 B1**

(12) **EUROPEAN PATENT SPECIFICATION**

(45) Date of publication and mention
of the grant of the patent:
**14.08.2019 Bulletin 2019/33**

(21) Application number: **11785483.6**

(22) Date of filing: **24.10.2011**

(51) Int Cl.:
*G06Q 10/00* (2012.01)     *G06Q 50/22* (2018.01)
*G16H 40/63* (2018.01)     *G16H 40/20* (2018.01)

(86) International application number:
**PCT/IB2011/054735**

(87) International publication number:
**WO 2012/059839 (10.05.2012 Gazette 2012/19)**

(54) **IN VITRO DIAGNOSTIC TESTING INCLUDING AUTOMATED BROKERING OF ROYALTY PAYMENTS FOR PROPRIETARY TESTS**

IN-VITRO-DIAGNOSETESTS MIT AUTOMATISIERTER VERMITTLUNG VON LIZENZGEBÜHREN FÜR GESCHÜTZTE TESTS

TEST DE DIAGNOSTIC IN VITRO COMPRENANT LE COURTAGE AUTOMATISÉ DE PAIEMENTS DE ROYALTIES POUR DES TESTS PROPRIÉTAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.11.2010 US 408684 P**

(43) Date of publication of application:
**11.09.2013 Bulletin 2013/37**

(73) Proprietor: **Koninklijke Philips N.V.
5656 AE Eindhoven (NL)**

(72) Inventors:
• **VAN DER ZAAG, Pieter, Jan
NL-5656 AE Eindhoven (NL)**
• **DIMITROVA, Nevenka
NL-5656 AE Eindhoven (NL)**
• **VAN DE STOLPE, Anja
NL-5656 AE Eindhoven (NL)**
• **VAN HOUTEN, Hendrik
NL-5656 AE Eindhoven (NL)**

(74) Representative: **Steffen, Thomas
Philips Intellectual Property & Standards
High Tech Campus 5
5656 AE Eindhoven (NL)**

(56) References cited:
**WO-A1-2004/044236     WO-A2-01/26029
WO-A2-2004/074456     WO-A2-2008/067551**

• BJORLING ERIK ET AL: "A web-based tool for in silico biomarker discovery based on tissue-specific protein profiles in normal and cancer tissues", MOLECULAR & CELLULAR PROTEOMICS, AMERICAN SOCIETY FOR BIOCHEMISTRY AND MOLECULAR BIOLOGY, INC, US, vol. 7, no. 5, 1 May 2008 (2008-05-01), pages 825-844, XP009160837, ISSN: 1535-9476, DOI: 10.1074/MCP.M700411-MCP200 [retrieved on 2007-10-03]
• REPSILBER DIRK ET AL: "Biomarker discovery in heterogeneous tissue samples -taking the in-silico deconfounding approach", BMC BIOINFORMATICS, BIOMED CENTRAL, LONDON, GB, vol. 11, no. 1, 14 January 2010 (2010-01-14), page 27, XP021065620, ISSN: 1471-2105

## Description

FIELD OF THE INVENTION

The following relates to the medical arts, medical diagnostic and clinical arts, clinical decision support (CDS) system arts, and related arts.

BACKGROUND OF THE INVENTION

Medical diagnostic and clinical practice is making increasing use of biomarker assays, driven by the increasing affordability of DNA and RNA sequencing systems, microarray technologies, and so forth. Some examples of in-vitro diagnostics biomarker assays include: DNA tests that detect the presence of a DNA mutations (where "mutation" is to be understood as encompassing structural variations and indels, that is, insertions or deletions); detection of a mutation that has been linked to a medical condition, and thus contains a clinical claim; microarray tests that detect DNA mutations and variations and the presence and/or concentration of a biomarker such as a DNA, RNA, methylated DNA, protein, or other biological "marker" molecule in a sample, or so forth. A biomarker may also comprise of a list of biologically significant entities with associated decision rule (or a computer based classification system) that classifies a sample based on the input levels of the set of these biological measurements into one of the possible categories (e.g. tumor vs. normal). To be the basis of a clinical claim, the biomarker assay is validated or certified by a qualified entity such as a medical association or a governmental regulatory entity (for example, the Food and Drug Administration, FDA, in the United States) on the basis of medical studies performed under medically accepted testing. In some situations, only one, or a few, laboratories are qualified or certified to perform the test.

Biomarker assays are available for routine medical use. In one approach, a dedicated testing "kit" is provided which includes all test containers, chemicals, or other apparatus utilized in performing the biomarker assay. In another approach, a sample is sent out to an external laboratory which performs the test (or a suite of tests) and reports the result(s) to the hospital. In yet another approach, an in-house sequencing apparatus is located at the hospital, and generates a complete genetic sequence for a portion of DNA or RNA, or even a generates a whole DNA genome, which then serves as the input data for one or more biomarker assays.

The increasing usefulness of biomarker assays in medical diagnostic and clinical practice is a consequence of the increasing availability of the requisite testing equipment (e.g., kits, DNA sequencing apparatus, microarray technologies, or so forth), and the synergistic development of biomarker tests for various medical conditions. Typically, a biomarker test is the result of painstaking medical research and development performed to identify, quantify, and validate an inference of a medical condition (for example, a particular type of cancer and possibly its stage in the patient) based on the presence/absence and/or level of a detectable biomarker or specific set of biomarkers. Where a set of biomarkers are used in the test, the clinical inference operates in a multidimensional space, which may span up to tens of parameters. The test development may also entail other components, such as developing a robust and practical biomarker detection mechanism (for example, a fluorescent marker that bonds with the biomolecule of interest to enable characterization by fluorescence), and determining possible sources of "false positives", that is, other (possibly benign) causes for the biomarker to appear and/or elevate in level. In most countries, the test development ultimately culminates in passing through a governmental approval process. In the United States, the approval process is under the jurisdiction of the Food and Drug Administration (FDA), which imposes stringent protocols for approval of any new diagnostic or clinical test. Even after initial development, the test may continue to be refined as additional clinical feedback becomes available.

The development and maintenance or updating of biomarker tests or other in-vitro diagnostic tests should be reimbursed in order to encourage research entities to continue and expand work in these areas. Conventionally, reimbursement is through the sale of dedicated test kits, or by laboratory fees if the test developer performs the test at an external laboratory. However, test kits can only be used if they are available, and the kits may have a limited shelf life. If a test kit, or a detection device that is needed for the readout of the test result, is not available, then the biomarker test must be delayed until a test kit (or detection device) is obtained. Using an external laboratory to run the biomarker test also introduces delay or the potential for a loss of the sample or a mix-up of samples from different subjects. In some instances, such as an aggressive cancer, these delays may be clinically problematic or even life-threatening.

Hospitals or other medical care providers may prefer to run the biomarker test locally using general-purpose equipment such as an in-house genome sequencer or on-site microarray laboratory. Such an instrument can be used to perform requisite DNA and/or RNA sequencing in-house in order to perform a surrogate biomarker test that is medically equivalent to the standard test performed by a certified, FDA-approved, or otherwise-qualified laboratory. Performing such a surrogate in-vitro diagnostic test in-house will typically reduce or eliminate delays, and may also be less costly. However, the test developer (or, more generally, test owner) is less likely to be reimbursed for locally run tests that do not use a test kit. Indeed, unless the biomarker test is patented by the developer, licensed to the hospital under a limited disclosure agreement, or is in some other way proprietary, the hospital has no legal obligation to reimburse the test owner for the surrogate equivalent test run on the hospital's own equipment. Even where reimbursement is owed, there is no convenient mechanism by which the hospital can identify and make such reimbursement.

Ready availability of an in-house genomic sequencer is also expected to expand the repertoire of conveniently available tests. Previously medical personnel might restrict the in-vitro diagnostic tests performed on a given patient to those tests specified by the applicable clinical guideline. However, the availability of a complete DNA sequence (or other extended genomic sequence, e.g. an RNA sequence) provides a vast quantity of data. For example, some DNA sequences may provide around 100,000 nucleotide base locations. This plethora of data can be expected to encourage medical personnel to consider running other genetic tests, including tests that may fall outside the applicable clinical guidelines, based on the physician's intuition, recent reports in medical journals that have not yet been incorporated into the clinical guideline, or other rationales. Indeed, it is even possible that medical personnel may discover an unexpected probative genetic mutation (or combination of mutations) in the patient's genomic sequence for which the medical personnel were not searching. In such cases medical personnel are even less likely to recognize whether the performed diagnostic test is subject to a royalty reimbursement - indeed, they may not even realize that such a test has been "performed".

Inadequate reimbursement of the test developer can have substantial adverse consequences, including lost revenue for the test developer and reduced incentive for developing, maintaining, and disseminating biomarker tests. This, in turn, may lead to the development of fewer new biomarker tests, increased "hoarding" of biomarker tests as trade secrets (for example, made available only through proprietary external laboratories or data analysis centers run by the test developer), and consequent reduction in quality and efficacy of patient care and higher medical costs.

While the foregoing has considered primarily biomarker tests as illustrative examples, more generally various in-vitro diagnostic tests that may be performed that may be subject to patent licenses, licensed trade secret information, or so forth. Similar concerns about inadequate reimbursement pertain to these types of proprietary procedures as well.

WO 2004/044236 discloses a method of determining the status of a subject by obtaining subject data and comparing the subject data to predetermined data which includes an indication of a condition.

WO 2008/067551 discloses a method of determining a Genetic Composite Index by assessing an association between an individual's genotype and at least one disease of condition.

WO 01/26029 discloses a system and method for securely providing personal clinical profile information to individuals over the Internet. The following provides new and improved apparatuses and methods as disclosed herein.

SUMMARY OF THE INVENTION

In accordance with a disclosed aspect, a method comprises: acquiring an extended genomic dataset, which dataset includes data indicative of or relating to an extended portion of a genome in which the data are not functionally unified; processing the extended genomic dataset in silico to discover at least one clinically significant feature of the dataset wherein the processing uses at least one of (1) a clinical guideline or decision tree and (2) nucleotide variant information retrieved from one or more genomic databases; and generating a clinical result based on the discovered at least one clinically significant feature of the dataset; wherein the processing and generating are performed by one or more computers.

The dependent claims define advantageous embodiments.

The advantages will be apparent to those of ordinary skill in the art upon reading and understanding the following detailed description.

BRIEF DESCRIPTION OF THE DRAWINGS

| | |
|---|---|
| FIGURE 1 | diagrammatically shows a medical diagnostic tracking system including a license brokering sub-system as disclosed herein. |
| FIGURE 2 | diagrammatically shows an illustrative royalty calculation according to an illustrative weighted royalty formula. |
| FIGURES 3 and 4 | diagrammatically show a process flow suitably performed using the system of FIGURE 1 including the clinical decision support (CDS) components. |
| FIGURE 5 | diagrammatically shows an illustrative display example corresponding to the step S5 of FIGURE 3. |
| FIGURE 6 | shows a process flow suitably performed by the system of FIGURE 1 when implementing a clinical guideline or decision tree in-silico using the CDS system components and operating on an extended genomic dataset. |
| FIGURE 7 | diagrammatically shows operation of the system with integral license brokering of FIGURE 1. |
| FIGURE 8 | diagrammatically shows a variant medical diagnostic tracking system including a license brokering sub-system disclosed herein, in which automated biomarker testing is integrated with license bro- |

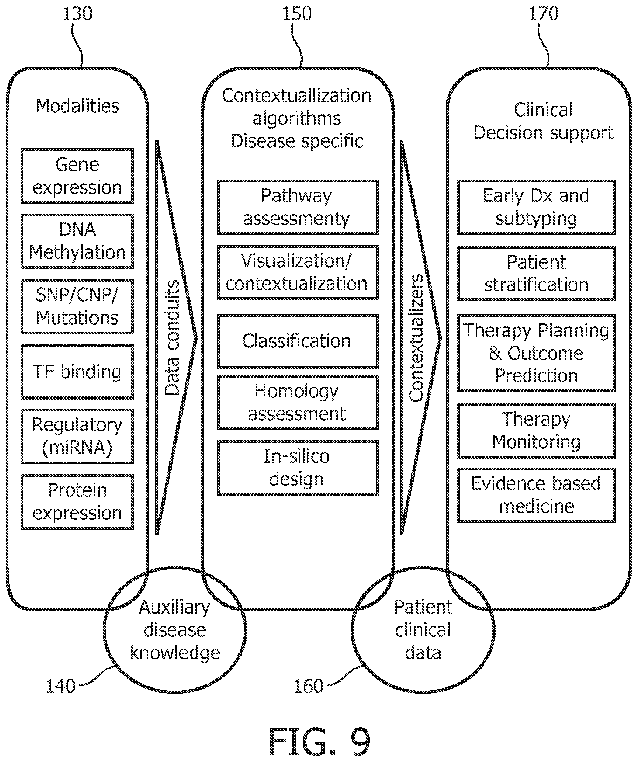

FIGURE 9 diagrammatically shows various components of biomarker testing and clinical decision support (CDS) operations which may be subject to license compensation suitably brokered by techniques disclosed herein.

## DETAILED DESCRIPTION OF EMBODIMENTS

It is recognized herein that fidelity to proprietary interests in biomarker testing or other in-vitro diagnostic tests, including the timely payment of any royalties, is in the interest of all parties. The patient (or, more generally, subject, e.g. also encompassing a screening subject, an outpatient, or so forth) benefits from prompt availability of useful proprietary in-vitro diagnostic tests. While the patient (or his or her insurance company) may have the burden of paying the license royalty as part of the medical costs, this expense is likely to be offset by avoiding more expensive alternative diagnostics or procedures such as exploratory surgery. The medical care provider also benefits from fidelity to proprietary interests of the in-vitro diagnostic test owner. Performing a proprietary procedure without authorization and (if required) royalty payment can result in substantial financial liability to the medical facility and/or the physician personally. On the other hand, the cost of a timely royalty payment can be passed on to the patient or his or her insurance company as a proper medical expense, and is not a cost to the medical care provider. The owner of the in-vitro diagnostic test directly benefits from receiving timely royalty payments, and may receive additional benefits from cooperative association with medical care providers such as valuable feedback on performance or outcomes. The general public also benefits at least indirectly from fidelity to proprietary interests in in-vitro diagnostic tests, through increased medical research and development efforts leading to better testing or the like which in turn reduces medical costs.

However, it can be difficult to ascertain whether there are proprietary interests in a given procedure, especially if the procedure is performed "in-house" as is increasingly the case for biomarker tests due to availability of general-purpose equipment such as genome sequencers. Moreover, even if it is known that a given in-vitro diagnostic test is covered by a patent or other proprietary interest, the physician may find it difficult to determine whether a royalty payment is owed and, if so, how much is owed. Still further, proprietary interests may not be limited to published patents, but rather may take other forms such as access to an in-vitro diagnostic test held as a trade secret and made available under terms of a Non-Disclosure Agreement (NDA) which may include a royalty term, or so forth. As yet another consideration, in some cases the owner of a patented test may refuse to license the test, and instead may require that the test be performed by the patent owner's own laboratory on a fee-for-service basis, or may require that the test be performed using a kit supplied by the patent owner. In such cases, performing the patented test using sequencing data generated by an in-house sequencer may infringe the patent. (Indeed, in some jurisdictions it is conceivable that merely acquiring the relevant sequencing data might be deemed to constitute infringement).

Disclosed herein are approaches for enabling timely payment of royalties for the use of proprietary biomarker tests or other proprietary in-vitro diagnostic tests. The approaches leverage existing medical diagnostic tracking systems, such as electronic health record (EHR) systems or the like, with which physicians and other medical care providers are already familiar and utilize in conducting their medical practice. In some embodiments, the medical diagnostic tracking system includes a clinical decision support (CDS) component that provides clinical decision support information - in such embodiments, the disclosed royalty payment techniques can be integrated with the CDS components to identify the cost of any royalty for consideration by the patient in deciding his or her treatment. The optional CDS components may also be configured to identify surrogate tests that may (for some patients and in certain clinical circumstances) be a viable alternative to a licensed in-vitro diagnostic test. A surrogate test may be clinically equivalent, in that it provides the same positive or negative result as the licensed test (or, said another way, equivalent in a "black box" sense), or may be equivalent at finer detail, for example providing equivalent analog data or profile shape.

The surrogate test may deliver fully equivalent results compared to "standard" test, so that the surrogate test constitutes a replacement for the "standard" test, or the surrogate test may provide data that is likely to be equivalent, and hence possibly useful, but which would require additional clinical trials to obtain FDA approval (or other relevant approval such as inclusion in clinical guidelines, i.e. "clinical adoption"). As an example of a fully equivalent test, analyzing the whole genome DNA sequencing result to identify a mutation in the gene for K-Ras is fully equivalent to a PCR-based commercial K-Ras mutation test. The therapy may be chosen based on this outcome from the whole genome sequencing, because the tests are fully equivalent: in both case at a specified position in the DNA a specific nucleotide is replaced by the mutant nucleotide (said another way, precisely the same mutation is identified). On the other hand, an example of a test that is not fully equivalent is as follows. Analysing the whole genome mRNA profile, either by sequencing or by several microarray hybridization techniques, enables quantification of the mRNAs that were present in the sample. On the other hand, in an FDA-approved MammaPrint® test, which is commercially available via Agendia, 70 mRNA's are quantified by a dedicated microarray by experienced personnel in an Agendia reference lab exactly according to FDA-approved protocol, and based on the result the prognosis of the patient is determined. When this test is performed using another protocol than described in the file for FDA approval, the results may provide similar information but the clinical claim as to prognosis is not clinically valid (that is, it is not considered "approved" by the FDA).

In the disclosed approaches, the medical tracking system is augmented by a license brokering sub-system which identifies when the system records the performing of a licensed in-vitro diagnostic test for which license information is stored in a licenses database and computes license compensation due to a licensor for the performing of the licensed in-vitro diagnostic test based on royalty calculation information stored in the licenses database. In some embodiments, the license brokering sub-system also remits the computed license compensation to the licensor using a computerized remittance approach. Advantageously, the brokering of the license royalty is transparent to the physician, who is therefore able to concentrate on determining whether the biomarker test or other in-vitro diagnostic test is medically appropriate for his or her specific patient (or, more generally, subject). In this way, proprietary biomarker tests or other proprietary in-vitro diagnostic tests are made readily available for medical use without overt encumbrance by licensing particularities, while still ensuring that timely royalty payments are made.

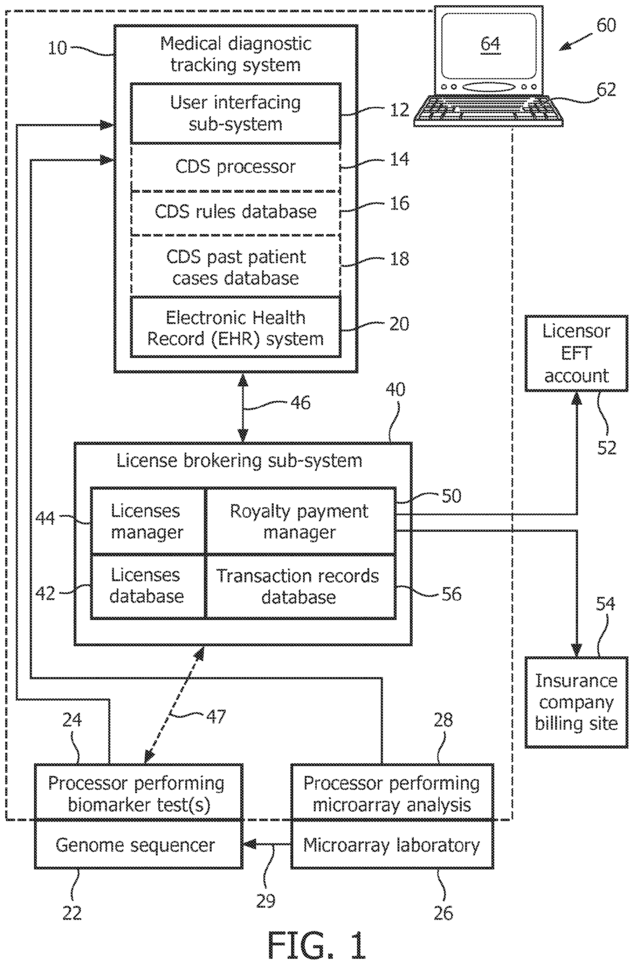

With reference to FIGURE 1, an illustrative medical diagnostic tracking system 10 is presented. A user interfacing sub-system 12 is provided by which a physician or other medical person inputs medical information that is tracked by the system 10. The user interfacing sub-system 12 may employ any suitable input interface. For example, a user-editable anamneses or patient case history form may be provided for the physician to fill out in consultation with the patient, including (by way of illustrative example): free-form text entry boxes for entry of patient information such as name, address, or so forth; drop-down lists, checkboxes, or so forth for constrained entry of self-reported patient symptoms, patient data (e.g., age, gender, or so forth), and other medical information; free-form text entry boxes for entry of medical "keywords" descriptive of the patient case or for entry of patient narratives self-reporting symptoms and/or case history; selection buttons for drilling down to more specific medical forms (e.g., a button may bring up a special form for oncology patients); or so forth.

In some embodiments, the medical diagnostic tracking system includes or has access to one or more clinical decision support (CDS) system components. A CDS system is a computerized system configured to link health observations with a database of health knowledge in order to provide patient case-specific information that may be useful to the physician and/or patient in making clinical decisions. In general, a CDS system is designed to assist medical personnel, but is not a substitute for the medical expertise of physicians or other medical personnel. The database of health knowledge, and its linkage with patient-specific health observations, can take various forms. In general, a CDS responds to a "clinical question" posed by the physician. The clinical question posed by the physician may be explicit, or may be implicit. As an example of the latter, the filled-out medical form may constitute the "clinical question", with the implied question being a request for a recommended course of assistance in clinical decision making for the patient.

CDS can be suitably implemented in various ways. The illustrative medical diagnostic tracking system 10 optionally includes or has access to CDS capability in the form of a CDS processor 14 which generates a response to the clinical question. The CDS processor 14 can employ or implement various algorithms or methods for generating the response. In some embodiments, the CDS processor 14 implements or utilizes a set of clinical rules that are stored in a CDS rules database 16. The clinical rules are suitably formulated by a selected group of medical personnel, with rules for a given clinical area preferably being formulated by one or more qualified medical experts in that area. Thus, the rules of the CDS rules database 16 embody the clinical expertise of these experts, and are based on the cumulative medical knowledge of the one or more qualified medical experts. The rules of the CDS rules database 16 are preferably formulated by medical personnel, but in some embodiments the actual coding of the rules may be performed by information technology (IT) personnel such as computer programmers or the like based on well established clinical evidence published by the panel of medical experts.

In some embodiments, the rules of the CDS rules database 16 may be formulated as "flow charts" or "decision trees" comprising sets of nodes interconnected by links. The current patient information places the patient at a current node (e.g., in the case of an oncology patient the current node may be chosen based on the type of cancer, its stage in the patient, and other pertinent information), and the links extending away from the current node represent possible clinical paths (or branches, i.e. decision paths of the decision tree) that the physician may follow. For example (again considering the illustrative oncology example), one branch may correspond to a radiation therapy regimen, another branch may correspond to a chemotherapy or antibody-based drug treatment regimen, or so forth. The response may then include displaying information about the current node and the various possible clinical paths (or branches) that the physician may follow. Such a response may be presented alone, so that the physician uses his or her judgment in deciding which path to follow, or may optionally further include a path recommendation generated by other rules of the CDS rules database 16. These rules can be derived as simple logic rules and linear dependencies, as well as derived from complex machine learning procedures involving feature classification and building classifiers using statistical learning methods (e.g. linear classifiers, Support Vector machines, neural networks, Hidden Markov Models, ensemble classifiers, to just mention a few).

Additionally or alternatively, the CDS processor **14** may access and utilize a CDS past patient cases database **18** in generating the response. In one suitable approach, a similarity metric is employed to compare the current patient case with cases in the database **18** to identify the most similar past patient cases in the database **18.** The similarity metric may include components measuring similarity of symptoms, number of common symptoms between the current patient case and the past patient case, or other items of similarity or dissimilarity. These most similar past patient cases may be presented to the physician, without further processing as part or all of the CDS response. In some embodiments, the CDS processor **14** may identify similar cases based on the genomic sequencing data such as DNA mutations, Single Nucleotide Polymorphisms, Copy Number Variations, indels, RNA-sequencing or Methylation-Specific sequencing. Single nucleotide variations (SNV) can be compared against known mutation markers which can be of various kinds including ones that cover important regulatory sequences such as exons, transcription factor binding sites as well as certain repetitive elements (e.g. Alu repeats). These can also include small insertions and deletions (indels). Copy number variations (CNV) can include deletions, amplifications, and various rearrangements (e.g. inversions) of whole genes or even larger genomic regions. Mutations of interest can also include variations of repeat elements such as SINE (breast cancer and Ewing's sarcoma have been linked with ALU insertions) and LINEs as well as microsatellites, minisatellites and macrosatellites. Methylation specific sequencing can reveal differential single nucleotide methylation changes (single C or A methylation) as well as aberrant genomic locus based (e.g. CpG islands) methylation changes. RNA sequencing can reveal changes both related to gene function, as well as regulatory RNAs and other non-coding RNAs (highly conserved). The similarity assessment can in general be based on any suitable amount of genomic data, ranging from only a few genes to data from the whole genome. Additionally or alternatively, the CDS processor **14** may further process the most similar past patient cases to identify in-vitro diagnostic tests that have been successful in similar past patient cases, or to identify other pertinent medical information (for example, negative information such as in-vitro diagnostic tests that have been unsuccessful in similar past patient cases), and this processed information may be presented to the physician as part or all of the response formulated by the CDS processor **14.**

The medical diagnostic tracking system **10** further includes or has access to an electronic health record (EHR) system or sub-system **20.** The EHR system **20** (also known in the art by similar phraseology such as "electronic patient record" or EPR or so forth) is employed to record medical care events including the performing of in-vitro diagnostic tests. The EHR **20** may be integrated with the medical diagnostic tracking system **10,** as illustrated in FIGURE 1, or may be a separate database system in communication with the medical diagnostic tracking system **10.**

In the course of providing medical care to a patient (or, more generally, subject), the physician may decide to perform a biomarker test or other in-vitro diagnostic test which is subject to a license. This decision may be made entirely by the physician (possibly in consultation with other medical personnel, reference to medical texts, or so forth), or may be made in consultation with clinical decision support information provided by the CDS component **14, 16, 18.** For example, the CDS **14, 16, 18** may recommend performing a licensed biomarker test as a patient diagnostic. Such a CDS recommendation may be explicit, or may take another form. For example, the CDS may display a clinical guideline that lists the particular biomarker test as a suitable diagnostic.

It is also contemplated that the decision to perform a biomarker test may be made spontaneously. This could occur "manually", in a "discovery" mode, in which the physician (or another qualified medical person) reviews the genome and identifies a clinically probative mutation for which the physician was not looking. Another way that a biomarker test could be performed spontaneously is in the case of clinical interpretation of the (extended) genomic data set by automatic processing to identify aberrant biological signalling pathways. In this situation a biomarker test could be performed that was not part of the clinical guidelines (that is, the clinical decision tree), but was performed by the biological pathway analysis tool, while a clinical decision was taken based on the pathway analysis result which now includes the patented biomarker. An illustrative example is analysis of cancer sequencing results of a patient with breast cancer, resulting in the in-silico performance of the methylated DMTC biomarker assay, which is associated with a clinical claim related to therapy resistance in colon cancer (and as such is clinically adopted). The treating oncologist of this patient could still use this result in the clinical decision making with regard to therapy to choose. (In this situation, the oncologist would not be making use of the colon cancer clinical claim.) Such a spontaneously discovered mutation (in the broadest sense, including aberrant DNA methylation) may in some jurisdictions be covered by a licensed patent.

Once a decision is made to perform the biomarker test (or other in-vitro diagnostic test), the test is carried out (or, in the case of a "discovery mode", the test is effectively performed simultaneously with the "decision" to perform the test). In some embodiments, the biomarker test is performed using an extended genomic dataset. As used herein, the term "extended genomic dataset" comprises a dataset that includes data indicative of or relating to an extended portion of a genome in which the data are not functionally unified. By way of illustrative example, an extended genomic dataset may comprise an extended DNA sequence, an extended RNA sequence, a protein profile indicative of proteins present in a tissue sample (e.g., acquired by mass spectrometry), or so forth. In some illustrative embodiments the biomarker test may be carried out on an extended genomic dataset acquired using an in-house genome sequencer **22.** The extended genomic dataset in this case may be partial (for example, sequencing a single chromosome or a portion of a single chromosome, or multiple selected genomic areas), or may include an entire genome, e.g. a complete DNA sequence for the subject. Alternatively, the sequencer **22** may be used to generate an extended genomic dataset comprising an extended RNA sequence. The genomic data are processed by a processor **24** configured to perform the biomarker test, for example by performing calculations as specified by the biomarker test using inputs comprising selected genomic information extracted from the complete or incomplete DNA genome (or from other genomic information acquired by the sequencer **22**). The combination of the sequencer and processor **22, 24** suitably defines a sequence-based biomarker testing apparatus. If the genome sequencer **22** is a general-purpose sequencer capable of generating a wide range of genomic data, then the testing apparatus **22, 24** can be readily configured to perform a wide range of sequence-based biomarker tests.

As another illustrative example of a biomarker test, in some embodiments the test may be carried out on an extended genomic dataset comprising a microarray dataset acquired using an in-house microarray laboratory **26** in combination with a processor **28** configured to perform microarray analysis. The term "microarray" as used herein is intended to be broadly construed as encompassing various in-vitro diagnostic arrays for genomic or other types of samples, such as by way of illustrative example a protein array, a mixed genetic/protein array, or so forth. The microarray may include test cells providing quantitative information about the expression of various biomarkers, and the processor **28** can be configured to perform various microarray-based biomarker tests by being programmed to perform calculations as specified by the biomarker test using inputs comprising selected information extracted from a microarray assay. The combination of the microarray laboratory and processor **26, 28** suitably defines a microarray-based biomarker testing apparatus. If the microarray laboratory **26** has a sufficiently wide range of testing capabilities, then the microarray-based testing apparatus **26, 28** can be readily configured to perform a wide range of microarray-based biomarker tests. Moreover, the microarray-based testing apparatus **26, 28** may also or alternatively be used to provide genome enrichment arrays **29** providing enriched genomic material that serves as input to the genomic sequencing testing apparatus **22, 24.**

In other embodiments (not illustrated), a surrogate protein biomarker test may be performed using mass spectrometry. In this approach, a tissue sample containing proteins is analyzed by a mass spectrometer which breaks the proteins down into peptide chains of various sizes. The resulting fragmentation spectrum observed by the mass spectrometer can be compared with known reference spectra to identify various proteins in the sample. Specific chemical modifications of proteins that are associated with activation (e.g. in case of a kinase) or changed function in the cell can be similarly identified given suitable reference spectra. The proteins (and chemical modifications thereof, such as phosphorylation, ubiquitination, acetylation, methylation, or so forth) identified by mass spectrometry can be used as a surrogate for a patented assay (for example an activated kinase, or Her2 overexpression associated with breast cancer).

In some embodiments, the acquisition of an extended genomic dataset is leveraged by combination with a clinical guideline (for example, implemented by the CDS components **14, 16, 18** that performs one, two, three, or more biomarker tests using the same extended genomic dataset in accordance with a process path through a decision tree or guideline. These embodiments take advantage of the ability to perform biomarker tests "in-silico", that is, in software, operating on the extended genomic dataset and with complex bioinformatics and statistical analysis. For example, the paths followed through the guideline or decision tree may dictate that if a first test generates a positive result then a second test should be performed, whereas if the first test generates a negative result then a third test should be performed. The results of the subsequent second (or third) test may in turn determine further paths of the guideline or decision tree to follow. In such an approach the ultimate path through the guideline or decision tree that is actually followed is not predetermined, but rather is determined dynamically based on the test results, and may therefore generate an unexpected (i.e., spontaneous) decision to perform a biomarker test.

Data acquired by the biomarker testing facilities **22, 24, 26, 28** including the test results, and optionally also including the underlying data such as the extended genomic dataset, and optionally also including derived data such as confidence intervals or the like, is input into the tracking system **10** for recordation in the EHR **20.** The entry of this data into the tracking system **10** may be fully automated, semiautomated, or manual. If manual or semiautomated entry is employed, then the user interfacing sub-system **12** may be utilized to receive manual inputs. Additionally or alternatively, some or all types of data may be input automatically by a link between the various data generating elements (e.g., the biomarker testing facilities **22, 24, 26, 28**) and the medical diagnostic tracking system **10.**

With continuing reference to FIGURE 1, in some instances the biomarker test (or, more generally, in-vitro diagnostic test) which is performed may be a proprietary in-vitro diagnostic test that is licensed to the medical facility and under the terms of which license a royalty payment may or may not be owed to the licensor. To accommodate such situations in a manner that is transparent to the physician or other medical personnel, the medical diagnostic tracking system **10** is augmented by a license brokering system **40** which includes a licenses database **42** containing information about licensed in-vitro diagnostic tests. Typically, this information includes at least royalty calculation information for each licensed in-vitro diagnostic test which is sufficient to compute compensation due for performing the licensed in-vitro diagnostic test. A licenses manager **44** employs the royalty calculation information to calculate the royalty payment owed (which may in some cases be zero) for a given performance of the licensed in-vitro diagnostic test.

The license brokering system **40** is linked with the medical diagnostic tracking system **10** by a communication link **46,** which may by way of illustrative example take the form of a software "plug-in" or the like. In some embodiments the communication link **46** takes the form of the license brokering system **40** being an integral part of the medical diagnostic tracking system **10.** By way of the communication link **46,** the license brokering system **40** considers each performed in-vitro diagnostic test as it is recorded in the EHR **20,** in order to determine whether the performed in-vitro diagnostic test is a licensed in-vitro diagnostic test. In one suitable approach, the EHR **20** employs a medical care event indexing system in which medical care events are uniquely indexed. In this case, each in-vitro diagnostic test has a unique index number. If the licenses database **42** stores this unique index number for each licensed in-vitro diagnostic test, then the identifying of a licensed in-vitro diagnostic test is suitably performed by comparing the index number of each performed in-vitro diagnostic test as it is recorded in the medical diagnostic tracking system **10** with the index numbers of the licensed in-vitro diagnostic tests - if a match is found, then the performed in-vitro diagnostic test is identified as the licensed in-vitro diagnostic test having the same index. In some such embodiments, the Current Procedural Terminology (CPT) code set promulgated by the American Medical Association is used as the set of unique index numbers for uniform identification of in-vitro diagnostic tests. This is merely an example, and the identifying of licensed in-vitro diagnostic tests can be done by other approaches, such as word description matching (assuming standardized word descriptions are used in recording performed in-vitro diagnostic tests in the EHR **20**).

In some embodiments, the medical diagnostic tracking system **10** includes identification of all available in-vitro diagnostic tests used at the medical care facility, or all available in-vitro diagnostic tests that have approval or have been adopted by the governing jurisdictional entity (e.g., the Food and Drug Administration or FDA in the United States). In general, some, but not necessarily all, of these in-vitro diagnostic tests are proprietary and subject to some licensing which may or may not include a royalty term. In some embodiments, the medical diagnostic tracking system **10** includes links or other associations between medically equivalent tests, so as (by way of illustrative example) to determine whether a substitute or surrogate in-house test may be employed in conjunction with a suitable royalty payment. For example, the tracking system **10** may identify a biomarker test that is normally performed by an outside laboratory -- but, if there is a medically equivalent licensed in-house test that can be performed using the in-house genome sequencer system **22, 24,** then the in-house test may be substituted and recorded when performed, and the license brokering system **40** then identifies the substituted in-house biomarker test during the recording as a licensed in-vitro diagnostic test.

If the license brokering system **40** identifies a performed in-vitro diagnostic test as a licensed in-vitro diagnostic test during the recording, then the licenses manager **44** computes the license compensation due for performing a licensed in-vitro diagnostic test based on the royalty calculation information for the performed licensed in-vitro diagnostic test stored in the licenses database **42.** In general, whether a royalty payment is owed, and if so how much, is determined by the terms of the license. This is codified in the licenses database for each licensed in-vitro diagnostic test in a manner enabling the licenses manager **44** to compute the license compensation due.

In one illustrative example, the license specifies the compensation due (i.e., the royalty) in terms of a royalty basis and a royalty rate. The royalty rate is the royalty amount due if the royalty basis is satisfied. For example, the royalty basis may be a positive result for performing the licensed biomarker test. In this case the licenses manager **44** computes compensation due in the amount of the royalty rate if and only if the licensed biomarker test is performed and yields a positive result. If a negative result is obtained, then the licenses manager **44** computes that no compensation is due (or, said another way, the compensation due is zero).

As another example, the royalty basis is merely the performing of the licensed biomarker test (regardless of the result). In this case, the licenses manager **44** computes compensation due in the amount of the royalty rate anytime the licensed biomarker test is performed, regardless of whether the result is positive, negative, or inconclusive.

In another contemplated variant, the royalty basis is the collection of genomic data that is used in the biomarker test. In this case, the licenses manager **44** computes compensation due in the amount of the royalty rate anytime the genome sequencer **22** acquires the relevant genomic data, regardless of whether or not the biomarker test is actually performed. In a variant embodiment, the sequencing system **22, 24** may access the licensing sub-system **40** prior to performing sequencing to compare a scheduled sequence with licenses that are filed in the licenses database **42.** This is diagrammatically indicated in FIGURE 1 by the arrow **47** shown in phantom in FIGURE 1. If the scheduled sequence includes a gene for which a royalty payment is required when sequencing that gene, the operator may be notified of this and asked whether sequencing of that gene should be omitted. In this way targeted sequencing can be performed that avoids generating extraneous genomic data requiring a royalty payment. (Note that for this embodiment it may also be useful to store information in the licenses database **42** pertaining to genes that are patented but not licensed, so as to avoid inadvertently sequencing and/or utilizing these patented genes).

As another example, the license may specify a fixed royalty payment amount that becomes due if and when the licensed biomarker test is first performed, with no further royalties due thereafter for subsequent performances of the biomarker test. In some such licenses, the fixed royalty payment may cover only a certain time interval, e.g. one year. Here the licenses manager **44** computes the compensation due as the fixed payment amount if the performance of the licensed biomarker test satisfies the fixed payment trigger condition (e.g., is the first performance of the biomarker test in the time interval of interest); otherwise, it computes the compensation due as zero.

As another example, the license may specify a fixed royalty payment covering a time period which allows unlimited use of the licensed biomarker test. In this case, the royalty calculation information stored in the licenses database **42** for the biomarker test is a fixed payment indicator that informs the license brokering sub-system **40** of the fixed royalty payment system. In this case, the licenses manager **44** computes the license compensation due for performing the licensed biomarker test as zero. (This assumes that the fixed royalty payment is made on an annual or other basis and is controlled, not by the license brokering sub-system **40,** but rather by some other accounting system as a fixed expense of the hospital or other medical care facility.)

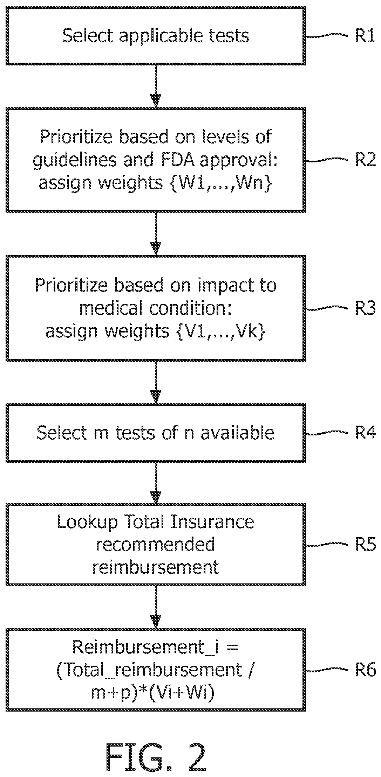

With reference to FIGURE 2, in another example the royalty calculation employs a weighted formula in which the royalty information includes a royalty percentage and an agreed weight, where the weight is based in part on clinical information provided by the CDS system **10.** In the illustrative example of FIGURE 2, a CDS operation **R1** determines a set of (e.g., n) available tests. The operation **R1** may be based, for example, on clinical guidelines provided by the CDS. In an operation **R2,** a first weight (W) is assigned based on a prioritization metric such as approval or clinical evidence as recommended by the panel of experts. The weight used in the operation **R2** is suitably embodied as a quantitative acceptance metric such as level of FDA approval or NCCN or ASCO recommendation or so forth stored in the licenses database **42.** In an operation **R3,** a second weight (V) is assigned based on the possible impact with respect to the clinical question. For example, the panel of experts may recommend that for certain medical conditions, it is preferred to have companion diagnostics type of tests because they answer a very specific question. Also we should note that the same biomarker could be used for multiple purposes, so a marker should preferably be coupled to a clinical question at hand. Again, the weight (V) is suitably embodied as a quantitative metric (tied to the clinical question via a lookup table or the like) stored in the licenses database **42.** Optionally, additional or other weights may be included, such as based on the hospital preference for a provider or trust in the provider of the biomarker, or the difficulty of interpretation or so forth. In an operation **R4** a set of m tests are selected based on the weights (e.g., weights W and V determined in respective operations **R2, R3.** In an operation **R5,** the CDS system **10** automatically looks up the CPT code and the recommended insurance reimbursement for performing the licensed test by outsourcing to an external laboratory. If the test can be performed in-house (e.g., using the sequencer laboratory **22, 24**), then an operation **R6** computes the royalty due, which may take into account factors such as the weights W, V, and optionally a "discount" since the insurance reimbursement includes the cost of actually performing the test by the outside laboratory, whereas the royalty due for an in-house test does not include that cost. In illustrative FIGURE 2, the royalty due is computed by dividing the recommended insurance reimbursement ($R_I$) by the number of biomarker tests performed within the same sequencing run. Thus, the final royalty reimbursement for performing test $T_i$ in-house is

given by: $R_i = \left( \dfrac{R_I}{m + p} \right)(V_i + W_i)$ where $W_i$ and $V_i$

are the weights W, V, respectively, for the test $T_i$. Here it is understood that the sum of all the weights will add up to 1. The constant $p$ is added to the number of participants because the in-house laboratory 22, 24 may assess a charge to cover for the expenses incurred in providing the test service.

In general, a royalty reimbursement will be charged only if the test is actually performed (and, depending on the license, only if other conditions are met - for example, some licenses may stipulate royalty payment only if the test result is positive). This can be relevant if a second test is run contingent on the results of a first test. For example, two mutations: one at the beginning of a gene (disrupting a GATA binding sequence or a TATA box, or frameshift within the first exon) will have more importance on the respective function of the protein produced by that gene, than a mutation that is, e.g., on exon 3 of the gene that may slightly change the conformation and therefore reduce the function of the gene, but does not completely disrupt the function. If two such mutation tests are scheduled, the former test is applied first and if it matches, then the second test is skipped. This is suitably accommodated by invoking the license brokering sub-system **40** to pay royalty compensation only when the performance of the test is recorded, e.g. in the EHR **20,** since at that point it is known that the test has actually been performed. (In this regard, it should also be noted that a physician will generally have authority to override or ignore any recommendation by the CDS system components **14, 16, 18** to perform a given test - accordingly, the mere recommendation by the CDS to perform a test is not generally a suitable basis upon which to pay royalty compensation.)

The term "license" as used herein denotes any instrument under which a royalty or other compensation is owed to a licensor in order to use the licensed in-vitro diagnostic test. For example, a license may comprise a license of a patent. In this case, the license covers the patented biomarker test, and the royalty terms are defined by the license. In some instances the terms of the license may differ from the scope of the patent. For example, the claims of the patent may require a positive result for the test, but the license may nonetheless require a royalty payment anytime the test is run regardless of whether a positive result attains. Conversely, the claims of the patent may recite an execution of the test without requiring any particular result, but the license may require a royalty payment only if the test yields a positive result. In such cases, the license (and not the claim scope) governs the royalty payment owed to the licensor.

The term "license" as used herein is not limited to a patent license. For example, in a joint research agreement between the medical care provider and a research organization, a royalty payment may be required whenever biomarker test developed under the agreement is performed by the medical care provider, even if that biomarker test is not patented. Again, the license governs in such a case. Still further, a "license" as used herein is not limited to agreements in which the medical care provider is a direct signor. For example, the medical care provider may be a member of a consortium that agrees that its members will pay a royalty for a certain biomarker test, in which case the agreement of the consortium governs the royalty payment. In some jurisdictions, a license may be generated by action of the government. For example, the government authorize use of the patented in-vitro diagnostic test so long as a "reasonable royalty" is paid to the owner of the patent. In this case the license is defined by the governmental authorization, the licensor is the patentee, and the terms of the license are payment of the government-defined "reasonable royalty" whenever the test is performed.

The term "licensor" denotes the person or entity to whom the compensation is owed under the license. The licensor may be a patent owner, or may have some other proprietary interest. In the earlier joint research agreement example, the licensor would be the research organization to whom the medical care provider owes a royalty under the joint research agreement (again, even in the absence of any patent). As another (non-patent) example, the biomarker test may be maintained by its developer as a trade secret, and the medical care provider may owe a royalty under a non-disclosure agreement (NDA) under which the medical care provider performs the biomarker test while keeping the test confidential as per the NDA. In such a case, the NDA serves as the license and the test developer to whom the royalty is owed is the licensor.

With continuing reference to FIGURE 1, in some embodiments the license brokering sub-system **40,** in addition to computing the license compensation owed to the licensor (as performed by the illustrative licenses manager **44**), also remits the computed license compensation to the licensor, the remitting also being performed by one or more computers. Toward this end, the information about licensed in-vitro diagnostic tests contained in the licenses database **42** suitably further includes licensor information sufficient to remit compensation to the licensor of the licensed in-vitro diagnostic test, and a royalty payment manager **50** uses this information to remit license compensation to the licensor. The nature of the licensor information stored in the licenses database **42** depends upon the payment mechanism to be employed by the royalty payment manager **50.**

For example, in some embodiments and for some licensors, the remittance may be performed by the royalty payment manager **50** generating an electronic funds transfer (EFT) conveying the license compensation to the licensor. In such cases, the EFT is directed to the licensor based on the licensor information stored in the licenses database such as a bank identifier code (BIC), international bank account number (IBAN), licensor account number, or other suitable identification number or code identifying the licensor's EFT account **52.** In some such embodiments, an invoice is also sent to an insurance company **54** or other entity that should reimburse the hospital for the royalty payment.

In some embodiments and for some licensors, the remittance may be performed by the royalty payment manager **50** printing an electronic check for the license compensation made out to the order of the licensor based on the licensor information stored in the licenses database. In such cases, the royalty payment manager **50** constructs the electronic check based on licensor information stored in the licenses database, such as a legal name or "dba" of the licensor, along with suitable licensor address information. The electronic check may be printed and delivered by mail, or may be delivered electronically. Again, an invoice may also be sent to the insurance company **54** requesting reimbursement for the royalty payment.

In the foregoing examples, the hospital (or other medical provider) makes the royalty payment and is then reimbursed by the insurance company (or by the patient, if the royalty payment is not covered by insurance). However, in some cases it may be preferred to have the insurance company (or patient) directly pay the royalty. In such embodiments, the remittance may be performed by the royalty payment manager **50** generating and conveying an invoice for the license compensation to the subject of the performed licensed in-vitro diagnostic test or an insurance company contracted with said subject. In such cases, the invoice includes instructions to remit payment to the licensor constructed by the royalty payment manager **50** based on the licensor information sufficient to remit compensation to the licensor stored in the licenses database. The invoice is suitably sent to an insurance company billing site **54,** electronically or by printing the invoice and mailing it via regular mail. Optionally, a notification may be sent to the licensor notifying the licensor that the licensed test was performed and notifying the licensor of the arrangement for royalty payment.

Optionally, the royalty payment manager **50** also records the remittance in a transaction records database **56.** In some such embodiments, the transaction records database **56** is operatively connected with the hospital billing department or other care provider accounting system so that the remittance is automatically recorded in the billing or accounting system.

The medical tracking system **10** (optionally including the CDS components **14, 16, 18**) and the license brokering sub-system **40** are suitably embodied by one or more computers, such as an illustrative computer **60.** The user interfacing sub-system **12** suitably operates in conjunction with a keyboard **62,** mouse (not shown), or other user input device, and in conjunction with a display device **64** of the computer **60.** In the illustrative embodiment medical diagnostic tracking system **10** and license brokering sub-system **40** are embodied by the single illustrative computer **60,** which may be a personal computer, a network server or server cluster, distributed computing system, a network of computers collectively embodying a "cloud computing" system, or so forth. Alternatively, the medical diagnostic tracking system **10** and the license brokering sub-system **40** may be embodied by different computers or different (but intercommunicating) computer networks, computing clouds, or so forth. Still further, the functionality of the licenses manager **44** and the royalty payment manager **50** may be embodied by different computers (or, again, different computer networks, computing clouds, or so forth) either of which may or may not be the same computer as that embodying the medical diagnostic tracking system **10.** In a contemplated example of this latter variant, the licenses manager **44** and the medical diagnostic tracking system **10** may be embodied by one computer, and the royalty payment manager **50** may be embodied by a different computer such as a computer providing billing and/or accounting services for the medical care provider.

In embodiments which include the CDS components **14, 16, 18,** these components may optionally operate in conjunction with the license brokering subsystem **40** to provide the physician and patient with information about any licensing costs that may be associated with performing a licensed in-vitro diagnostic test. For example, the physician and patient may be informed if a given in-vitro diagnostic test has an associated license royalty that is not covered by the patient's insurance. The patient can then decide, in consulation with the patient's physician, whether or not to perform the test, or whether to select a surrogate test if one is available.

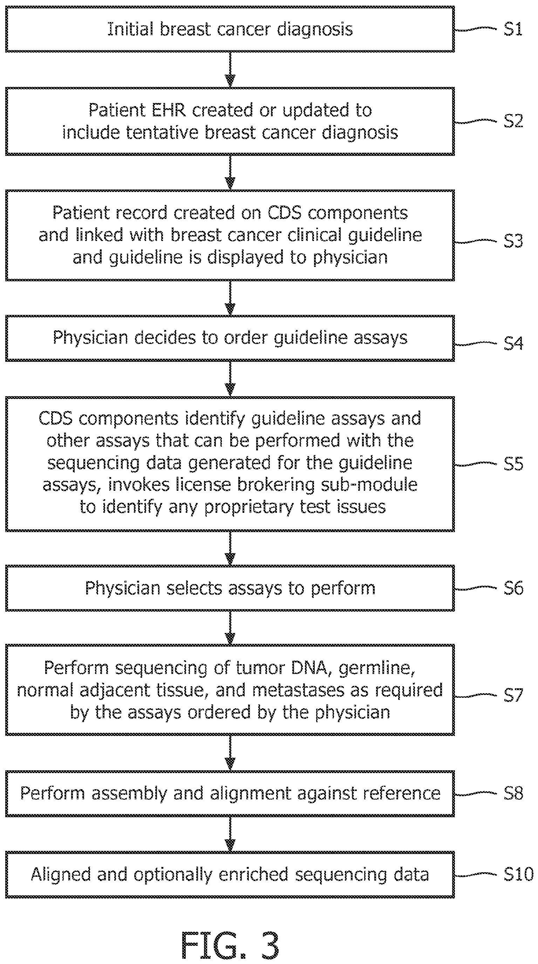

With reference to FIGURES 3-5, an illustrative example of synergistic operation of the CDS components **14, 16, 18** and the license brokering sub-system **40** is described. In a step **S1,** the physician makes an initial breast cancer diagnosis of a patient, and has all the clinical parameters for the patient, e.g. type of cancer, age, other relevant patient private data, other diagnostic tests, TNM classification of the malignant tumor (TNM denotes a cancer staging system characterizing the tumor "T", the regional lymph nodes "N", and the distant metastasis "M"), or so forth, input into the patient record in step **S2.** As this embodiment includes the CDS components **14, 16, 18,** a CDS record is also created and an appropriate clinical guideline (e.g., from the rules database **16**) is assigned in a step **S3.** Based on the guideline and/or the physician's medical expertise, the physician decides to order one or more guideline assays in a step **S4.** The CDS processor **14** retrieves from a database (e.g., rules database **16,** which has been assembled by a panel of experts) and presents a menu with biomarker options for further testing that the clinician can choose. These choices can include: biomarkers which are included in guidelines based on highest level of evidence for clinical utility, biomarkers which are available but not recommended in the guideline, and novel biomarkers which are at least published in the scientific literature. These may include mutations (or any other alterations that may influence gene function) of tumor suppressor genes, oncogenes, transcription factors, chromatin remodelling factors, and the like. These may also include published and experimentally well supported pathways (or gene networks) that are available for implementation. The CDS processor **14** identifies information about each available assay, so that the clinicians is fully cognizant of the risks. The CDS processor **14** also interacts with the license brokering sub-module **40** to determine any reimbursement implications.

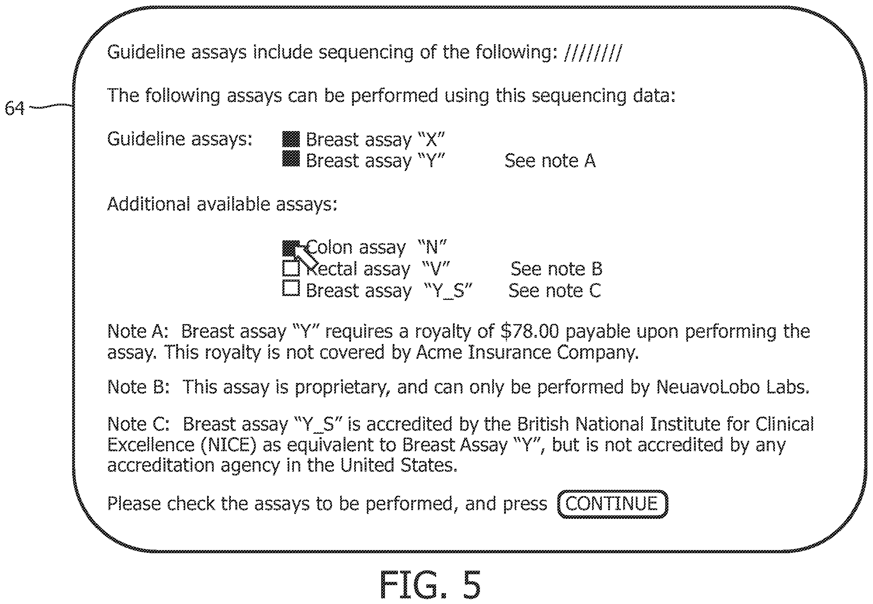

With brief reference to FIGURE 5, an illustrative display is shown. In this illustrative breast cancer example, the physician has ordered that the assays recommended in the guidelines be performed. As indicated in the first line of the display of FIGURE 5, performing these assays would entail sequencing a certain genomic portion (diagrammatically denoted in FIGURE 5 by "//////"). The guideline recommends two assays: "Breast assay 'X'" and "Breast assay 'Y'". (Note that the content of FIGURE 5 is a fictive example, and the assays and other mentioned entities are not intended to denote any actual assays or entities). Additionally, the genomic portion "//////" could be used to perform any of the additional (illustrative) three assays listed as additional available assays, namely: "Colon assay 'N'", "Rectal assay 'V'", and Breast assay 'Y_S'". The display further provides additional information about some of these available assays. "Note A" indicates that "Breast assay 'Y'", which is one of the guideline assays, is subject to a royalty fee of $78.00, which is not covered by the patient's insurance. "Note B" indicates that "Rectal assay 'V'" is a proprietary assay that has not been licensed, and accordingly can only be performed by NeuavoLobo Labs (an illustrative fictional example of an outside private laboratory). This is mainly informational - although in principle Rectal assay 'V' could be performed using the genomic portion "/////", doing so would infringe upon a patent owned by NeuavoLobo Labs, and accordingly this assay cannot be performed in-house. "Note C" indicates that "Breast assay 'Y_S'", which is not one of the guideline assays, is accredited in Britain as equivalent to the guideline assay "Y", but is not so accredited in the United States. In this example the physician is operating in the United States, and so "Breast assay 'Y_S'" is not recommended under the (U.S.) guideline. Nonetheless, the physician may consider ordering "Breast assay 'Y_S'" as a surrogate for the guideline-recommended "Breast assay 'Y'", especially in view of the royalty that would be due for performing "Breast assay 'Y'", which is not covered by the patient's insurance. The final decision is up to the physician, in consultation with the patient. In the example of FIGURE 5, the physician has selected the guideline assays, and has additionally selected to perform "Colon assay 'N'" (using the mouse pointer shown in FIGURE 5 to click on the selection box to the left of "Colon assay 'N'"). This latter selection is based on the physician's expertise, together with the knowledge provided by the CDS that this assay can be performed using the sequencing data that is already being acquired for performing the guideline tests - hence, "Colon assay 'N'" can be performed at low cost since it does not entail additional sequencing operations.

With returning reference to FIGURE 3, the physician orders the assays in step **S6.** The testing procedure starts with a sequencing step **S7,** which may be of a whole genome or of specifically enriched genomic areas. A next step **S8** is full assembly of the reads including alignment of the reads against a reference genome. This reference could be different based on the ethnic background of the individual. The assembly operation **S8** also performs all the appropriate data quality checks before any sequence alignment is performed.

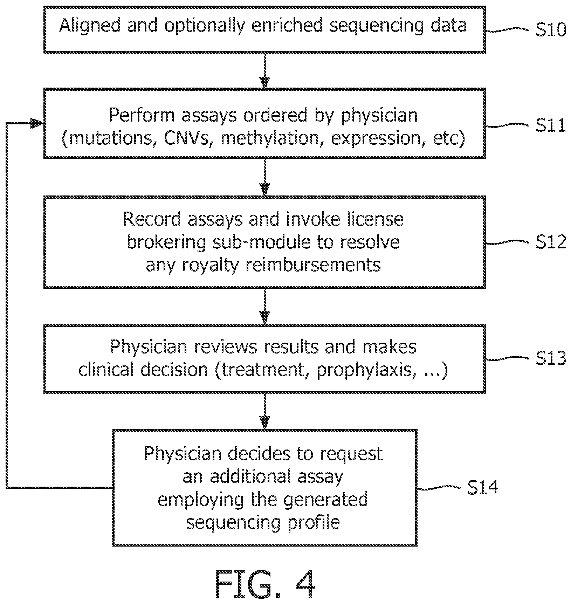

With reference to FIGURE 4 (which continues the flow diagram of FIGURE 3), the assembly operation **S8** produces aligned (and optionally enriched) sequencing data **S10.** In an operation **S11,** the system executes the appropriate assays based on clinician's instructions. The results are recorded in the EHR **20** in an operation **S12.** Additionally, the operation S12 invokes the license brokering sub-system **40** to identify, quantify, and optionally remit any royalty payments due for performing the tests. The physician reviews the results in an operation **S13,** and the physician makes a decision regarding treatment (or decides on further actions, e.g. prophylactic mastectomy). In an optional operation **S14,** the physician may order further tests, possibly utilizing the sequencing data **S10,** if additional probative information is desired by the physician.

In the process of FIGURES 3-4, the sequencing steps **S7, S8** are suitably performed by the in-house sequencing system **22, 24.** The performing of the assays (step **S11**) is performed by the in-house sequencing system **22, 24** and/or by the microarray laboratory **26, 28.** An exception is that if a test is proprietary and unlicensed, in which case it may need to be performed at the test owner's laboratory (or another laboratory that has an appropriate licensing arrangement with the test owner). In illustrative FIGURE 5, an example of this situation is "Rectal assay 'V'", which must be performed (if at all) by the outside laboratory NeuavoLobo Labs.

In the example of FIGURES 3-5, the biomarker tests are selected by the physician, possibly in an iterative fashion in which the results of one test lead the physician to order additional tests.

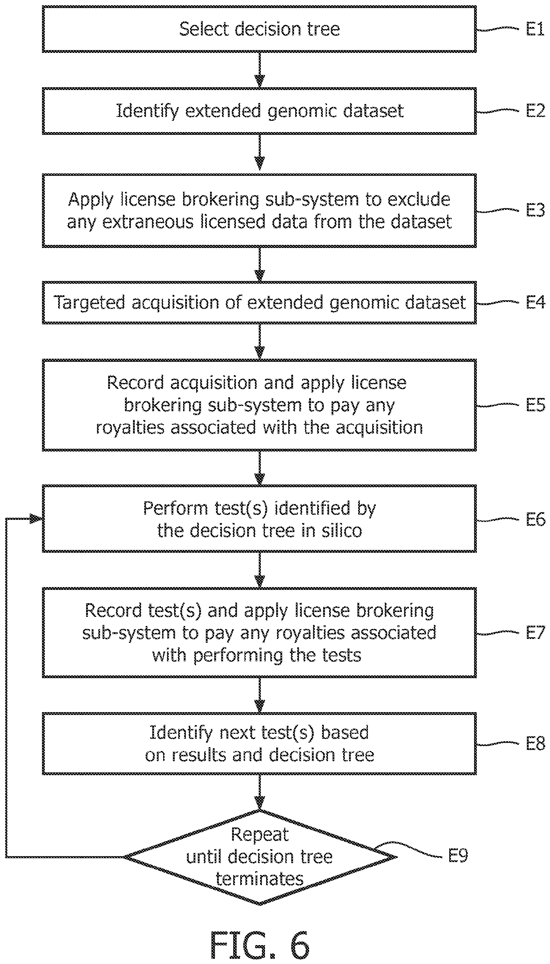

With reference to FIGURE 6, in another approach some or all such processing is automated by the CDS system **14, 16, 18** which performs test in accordance with a clinical guideline or decision tree that has been approved by a panel of experts, a guideline committee, or so forth, based on clinical studies or other evidence. In general, the clinical guideline or decision tree may represented by a graph model, a Boolean network, or so forth. The clinical guideline or decision tree may, for example, be stored in the CDS rules database **16** in the case of a simple graph model or the like, or the clinical guideline or decision tree may be embodied as a software module or component implemented or executed by the CDS processor **14.** Insofar as the CDS system **14, 16, 18** may in general include a number of available clinical guidelines or decision trees, the physician selects a clinical guideline or decision tree for use in an operation **E1**, for example, using an interface similar to that of FIGURE 5 but in which the guidelines are listed as selectable options. An extended genomic dataset including (but generally not limited to) the requisite data for performing the tests of the clinical guideline or decision tree is selected in an operation **E2.** In some cases this may be the entire genome, but in other cases it may be a part of a genome. Alternatively or additionally, the extended genomic dataset may include non-sequencing data such as microarray data or mass spectroscopy data. In an optional operation **E3,** the license brokering sub-system **40** is invoked (see path **47** in FIGURE 1) to identify and exclude from the dataset any extraneous data that is licensed such that its acquisition or processing would incur a royalty fee. In an operation **E4,** a targeted acquisition of the dataset (excluding any licensed data identified in the optional operation **E3**) is performed. In an operation **E5,** the acquisition of the extended genomic dataset is recorded in the recordation system **10,** and any royalty payment(s) due for acquiring the data are calculated (and optionally remitted to the licensor(s)).

Thereafter, the CDS processor **14** follows the clinical guideline or decision tree (selected in the operation **E1**) in-silico, meaning that the various biomarker tests indicated by the clinical guideline or decision tree are performed in software using the acquired extended genomic dataset (operation **E6**). In an operation **E7,** as each test is performed the results are recorded by the recording system **10** and the license brokering sub-system **40** is invoked to calculate (and optionally remit) any royalty payment that is triggered by performing the test or by the test results. (Note that any royalty payment that is triggered by the acquisition of the data was assessed at the dataset acquisition stage in the operation **E5**). In general, not all tests of all paths of the clinical guideline or decision tree will be performed - rather, only a subset of those tests are performed, with later tests being chosen dynamically based on results of previous tests in accordance with the branching defined by the clinical guideline or decision tree (operation **E8**). Processing continues until the clinical guideline or decision tree terminates (operation **E9**). The approach is advantageously fully automated once the extended genomic dataset is acquired.

It will also be appreciated that more than one clinical guideline or decision tree may be performed in silico using the same extended genomic dataset. For example, if the entire DNA genome is acquired as the extended genomic dataset, then any clinical guideline or decision tree consisting of DNA tests can be performed in silico using the acquired whole DNA genome.

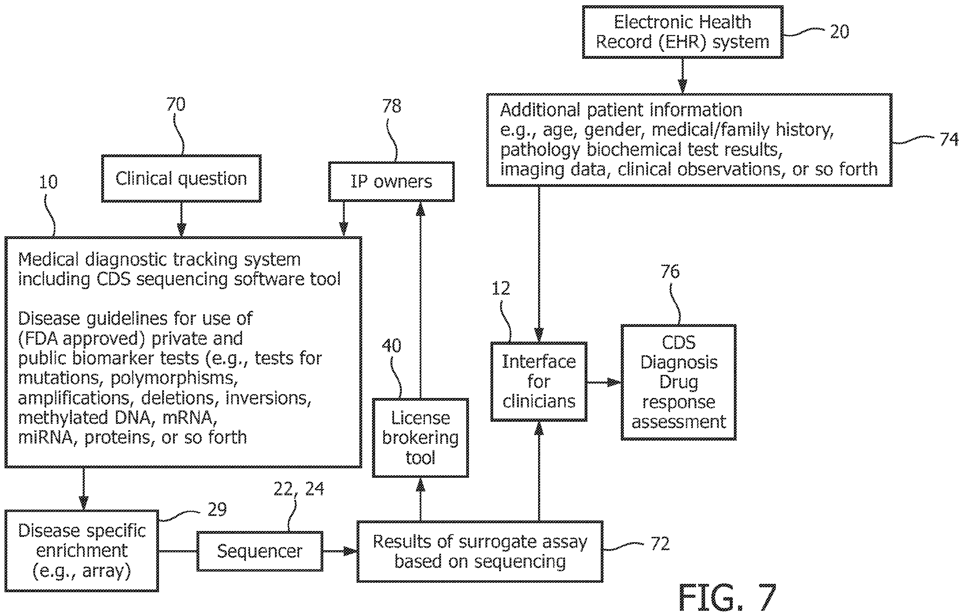

With reference to FIGURE 7, another embodiment is disclosed. In this embodiment, the medical diagnostic tracking system **10** includes a CDS sequencing software tool which is used to answer a specific clinical question/query **70**. In the illustrative example, a response to the query **70** would include recommending a certain biomarker test that is typically performed by an outside validated/FDA approved laboratory. However, the in-house sequencing system **22, 24** is capable of performing the requisite DNA and/or RNA sequencing to perform a surrogate biomarker test that is medically equivalent to the test performed by the validated/FDA approved laboratory. The answer to the clinical question **70** may also include recommendations with respect to the choice of genomic areas, or mRNAs to select (for example through genome enrichment arrays) for subsequent sequencing. In this case for the enrichment arrays, locally fabricated micro-arrays for genomic selection can be used (for instance, Flexgen technology, available from FlexGen BV, Leiden, The Netherlands) or standard genome enrichment arrays can be modified such that only the relevant probes for the selection questions are available for interpretation. Alternatively in-solution based methods may be used. This enrichment may thus be performed in hardware by genomic capture experiments of the required fragments. A recommendation for disease enrichment performed "in-silico" may also be provided. Such "in-silico" disease enrichment is performed by processing of the data using a set of filters that narrow down the number of genes or genomic loci that need to be interpreted.

Assuming that the physician accepts the recommendations of the CDS, the optional enrichment is performed **29** and the sequencer **22, 24** performs the DNA, RNA, or other sequencing so as to perform the surrogate biomarker test that is medically equivalent to the test performed by the certified laboratory. The results **72** of the surrogate biomarker testing based on the sequencing performed by the in-house sequencing equipment **22, 24, 26, 28,** along with other relevant information **74** from the EHR **20** or elsewhere (such as patient age, gender, medical/family history, pathology biochemical test results, imaging data, clinical observations, or so forth), are presented to a physician or other medical personnel via the user interfacing sub-system **12** of the illustrative medical diagnostic tracking system **10**. This information is used by the physician to develop a treatment regimen for the patient such as a diagnosis drug response assessment **76** supported by the CDS components **14, 16 ,18** of the medical diagnostic tracking system **10**.

In general, a number of individual PCR based tests serving as inputs for making the clinical decision responsive to the query **70,** which are typically performed by several different dedicated service providers, can be replaced by surrogate in-house tests. To avoid infringing on the proprietary rights of the test owners (i.e., licensors), the license brokering tool **40** provides reimbursement to intellectual property (IP) owners **78** who have licensed the biomarker tests (or, more generally, in-vitro diagnostic tests) to the hospital or other entity that performs the surrogate tests using the in-house sequencer **22, 24**.

Advantageously, the disclosed approaches reduce complexity for the hospital or other medical care provider by having one service, which deals with all the owners of the various biomarker assay IP (or other invitro diagnostic test licenses) and keeps the software tool **10, 40** updated with the latest results. For example, as licenses are added, updated, renegotiated, or so forth, this information is suitably updated in the licenses database **42** to ensure full compliance with new or updated royalty obligations. Such updating, when applied to one or more of the CDS components **14, 16, 18,** has the added advantage that new biomarker tests are brought to the attention of the physician by inclusion of the new or updated biomarker test licenses, thus ensuring that physicians are relying upon the latest biomarker testing capabilities. Similarly, updating might be performed with respect to additional studies and additional evidence support for the biomarker. For example, in the integrated CDS/royalty compensation example of FIGURE 2, the weights W, V of the biomarker licensing formulation may also be updated to reflect (for example) a recent FDA approval of the test $T_i$ which raises the corresponding weight $W_i$.

The integrated CDS and brokering systems **10, 40** can be embodied as a centralized system where both a web client and a server are both located at the service owner (e.g., the hospital via the illustrative computer **60**). In other embodiments, more ubiquitous usage is supported, in which there is a web client application that will be the front end to offer biomarker assay selection and accept user choices, while the storage, access and method execution, billing and IP license resolving is at a server or server cluster having the storage and computing power to execute thousands of queries simultaneously.

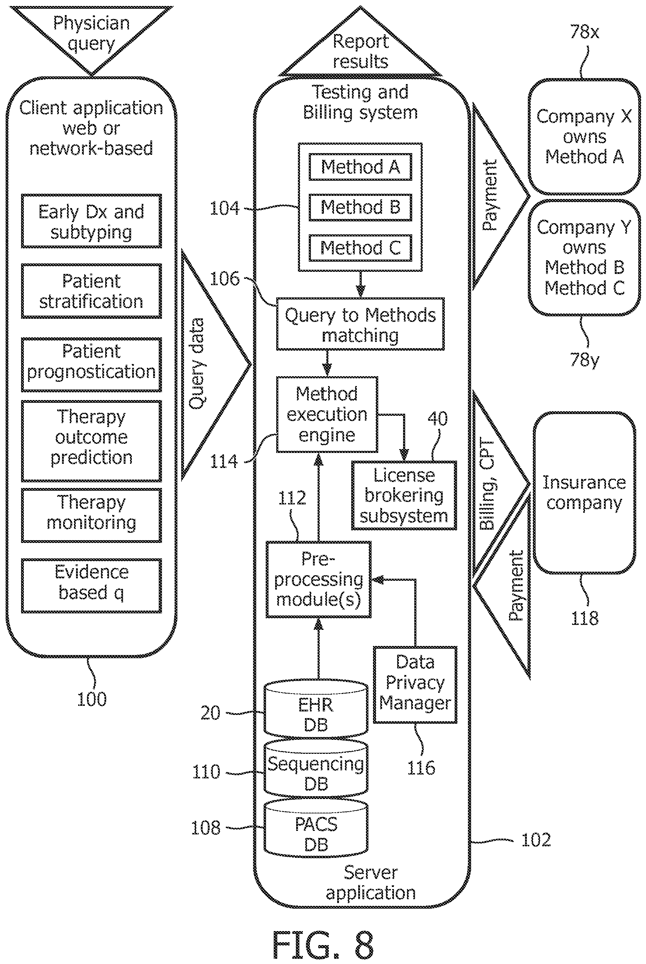

With reference to FIGURE 8, an illustrative clinical decision system 100 is suitably embodied as a web front end (or another type of light-weight software client). The clinician query is entered via a Web browser or an application running on a PDA/phone, optionally interactively while the system shows several options for the clinician to choose the appropriate test. In some contemplated embodiments, this is implemented using Simple Object Access Protocol (SOAP) which is an XML-based protocol to let applications exchange information over hypertext transfer protocol (HTTP). Alternatively, the front end could be implemented on a PDA/communication device such as a Blackberry or iPhone. A testing/billing component **102** performs both the biomarker testing (based on input such as a genomic sequence) and concurrent billing to compensate licensors of licensed biomarker tests. The testing/billing component **102** integrates functionality of the CDS components **14, 16, 18** and license brokering subsystem **40** of FIGURE 1, and contains a database 104 of methods for individual biomarker tests, which are implemented, tested and verified for correctness. In some embodiments, the methods of the database **104** are established to be medically equivalent to FDA-approved or otherwise validated biochemical assays performed by outside laboratories. Each test is executed by a corresponding method for which the IP rights are held by a licensor such as a company or academic institution or so forth. The system keeps a record of all licenses and is updated periodically with new or updated (e.g., renegotiated) licenses. A methods-IP update sub-module (not shown) updates the licensed in-vitro diagnostic tests and corresponding licenses. New or updated licensing information may be entered by suitable clerical personnel in consultation with the hospital legal department. It is also contemplated for updates to be acquired from an industry standards site such as the American Society of Clinical Oncology (ASCO) or the American Association for Cancer Research (AACR).

A test recommendation module **106** attempts to match all the clinical data of a certain patient, past guidelines, FDA approvals and recommend a battery of tests that are available in the system (or outside the system) that would be suitable for making therapy decisions for the patient. There could be multiple companion diagnostic tests that are applicable to the patient and they can be performed within the same sample sequencing. Such an approach is efficient if these multiple tests are using the same modality. The test recommendation module **106** takes the query and matches it to the appropriate test which is implemented by one or more biomarker methods (algorithm that embodies the in-silico equivalent of a biomarker in the sequencing data output).

The illustrative test recommendation module **106** optionally performs CDS functionality based on suitable clinical guidelines. Guidelines for recommending biomarker tests for use in clinical practice, such as those from American Society for Clinical Oncology (ASCO), the National Cancer Center Network (NCCN), the British National Institute for Clinical Excellence (NICE), or so forth, may be implemented by the recommendation module **106**. For example, through a rigorous systematic review of published evidence, ASCO produces Clinical Practice Guidelines, upon which summaries and practice tools are suitably based. The recommendation module **106** optionally receives information from the EHR **20** or other information sources for use in formulating recommendations. The recommendations may include recommending the performing of one or more biomarker tests to provide additional diagnostic information. The guideline recommendations can provide clinical decision support, information about dosing, and so forth. In some embodiments the guidelines are structured as decision trees and coded in XML or another suitable format. Recommendations related to drug classes are optionally tagged with ATC codes of the Anatomical Therapeutic Chemical (ATC) Classification System. Mapping may be used to couple the guideline with a drug database containing detailed information about each medication.

If the recommendation module **106** generates a recommendation to perform a biomarker test, this recommendation is typically reviewed by the physician or other medical personnel who actually authorize performing the biomarker test. The sequencing laboratory then performs the sequencing. In the integrated testing/licensing resolution approach implemented by the testing/billing component **102** of FIGURE 8, the biomarker test is performed automatically once the genomic sequence generated by the sequencer is input to the method-IP database component **102**. The biomarker test may optionally also utilize other information about the patient, such as patient data stored in the electronic health record **20** or in a Picture Archiving and Communication System (PACS) database **108** which stores medical images. After the sequencing, alignment is performed and the data is stored in a sequencing database **110** which could store genomic sequences from various modalities such as DNA-seq, RNA-seq, CGH-seq, Methyl-seq, or so forth.

One or more preprocessing modules **112** are optionally provided to perform functionality including modality specific preprocessing of the data. For example, an RNA pre-processor may be provided, by which RNA-seq data is analyzed for the presence of splice variants of the same gene and expression profiles. A DNA pre-processor may be provided, by which DNA sequencing data is processed to find single nucleotide polymorphisms (SNPs), rearrangements and other DNA changes. A comparative genomic hybridization (CGH) pre-processor may be provided, by which segmentation values are determined from sequencing data in order to provide copy number information about the genome. A Methyl-seq pre-processor may be provided, by which the resulting sequences are matched to the in-silico "bisulfite" converted genome and abnormally methylated nucleotides are recorded. Each of these modules **112** produces data that is preferably stored in an interchangeable format and linked to various annotation tables converted into a uniform format from various public repositories (such as NCBI, UCSC, Ensembl, or so forth), and optionally categorized into classes such as structural/functional, normal/disease-causing variations, or so forth.

The output of the optional one or more preprocessor modules **112** serves as input to a method execution engine **114.** The biomarker methods are suitably implemented using a computer programmed in a suitable programming language or environment such as Java, C, C++, C#, Perl, R/Bioconductor, Fortran, or so forth, and executes the appropriate biomarker test or tests using suitable input which (in addition to the sequencing data, optionally preprocessed) may include patient data, imaging data, or so forth obtained from various patient data information systems **20, 108.** The method execution engine **114** applies the appropriate method that evaluates the parameters in order to provide the assessment for the presence or absence of certain mutations, levels of RNA, SNPs, or so forth. The biomarker test may in some cases implement a complex set of decision-making rules and/or mathematical equations. Pattern or template recognition approaches, clustering methods, classification methods such as support vector machine (SVM) or hidden Markov model (HMM) approaches, survival analysis, Cox proportional hazards, or so forth, may be employed to determine the patterns in a disease-specific manner in which correlation to clinical information is also optionally integrated. Optionally, a visualization of the results of a particular biomarker test may be provided. For example, a Kaplan Meier curve may be shown for all the patients with similar biomarker profile, as an added visualization to aid in clinical decision making. The performing of the biomarker test is recorded in a suitable database such as the EHR **20** (data flow connection not shown in FIGURE 8).

A data security/privacy module **116** ensures suitable privacy protocols are applied when a query is posed from a clinical staff. These privacy protocols ensure that patient privacy and data security is maintained. For example, to maintain patient privacy in some embodiments, the data security/privacy module **116** ensures that clinical staff does not have access to the actual sequencing data, but rather sees only the quantified levels (such as gene expression levels or information about whether there are mutations that have clinical outcome implications or so forth).

The server-based method-IP database component **102** of FIGURE 8 includes the license brokering subsystem **40** which computes compensation due to licensor(s) if the biomarker test is a licensed biomarker test. The license reimbursement is computed according to the license terms as set forth in the licenses database **42** (for example, according the license reimbursement model of FIGURE 2, in some embodiments). The appropriate billing CPT code is used in computing the reimbursement to be paid (directly or indirectly) by an insurance company **118** or other paying entity. The CPT code set describes (among other things) diagnostic services and is advantageously designed to communicate the same type of information about healthcare services and procedures among physicians and insurance companies, as well as coders, patients, accreditation organizations. This illustrative approach is suitable in the United States, for which the Current Procedural Terminology (CPT) code set is maintained by the American Medical Association through the CPT Editorial Panel. Other countries typically have equivalent coding systems. The license brokering subsystem **40** references the appropriate licensing agreement (or equivalent content of the licenses database **42,** see FIGURE 1) to determine the compensation due the licensor **78x** and/or licensor **78y.** The payment manager **50** (again see FIGURE 1) of the license brokering subsystem **40** administers the payment (electronically, e.g. by EFT or printing an electronic check) to the licensor **78x, 78y**

Optionally, the CPT billing code and the type of procedure information is also used for administrative, financial, and analytical purposes. For example, the CDS components optionally maintain statistics about trends at a whole population with particular clinical parameters (for example, via the illustrative transaction records database **56,** see FIGURE 1). Such compiled information may be desired for compliance with government reporting requirements, or may be useful for companies which perform business-analytical services and would benefit from including the latest trends in their market reports.

Typically, the use of a given licensed in-vitro diagnostic test is covered by a single license having a single licensor. However, it is possible that a given licensed in-vitro diagnostic test may be covered by two or more licenses and/or two or more licensors. For example, in a dependent licensing arrangement, the method A of FIGURE 8 may include the method B. Accordingly, performing the method A requires payment of compensation due to Company X **78x** which owns method A and also payment of compensation to Company Y **78y** which owns method B. In another example, a single in-vitro diagnostic test may be co-owned by two or more licensors, each of which is owed compensation when the in-vitro diagnostic test is performed. Preferably, the license brokering subsystem **40** is configured to handle such situations, for example by using a data structure for the licenses database **42** that accommodates two or more licenses or two or more license compensation computations associated with a single licensed in-vitro diagnostic test.

With reference to FIGURE 9, some illustrative examples of clinical decision support (CDS) workflow suitably implemented in whole or in part by the CDS system **10** are set forth. The CDS may recommend, or base recommendations on results of, a set of data modalities **130** that may produce output using sequencers or other high throughput equipment such as: gene expression (RNA-seq or miRNA), DNA methylation (Methyl-seq), Single nucleotide polymorphisms, and Copy Number polymorphisms (CGH by sequencing), protein-DNA interactions such as transcription factors binding (ChIP-seq), protein expression, or so forth. Such modalities may employ licensed in-vitro diagnostic tests, e.g. licensed biomarker tests, for which the license brokering subsystem **40** ensures compensation due under the license is paid to the licensor.

In this case, certain DNA tests can be implemented by applying a classification model (for example, a Bayesian model, a Support Vector Machine model or other statistical learning model) derived from a previous clinical study (preferably a published and approved study). In such testing, it is contemplated that a new result may be observed, such as a gene or genetic locus that has not been seen before (e.g. a known gene ARID1A but now in a context of glioblastoma), or a panel of well known tumor suppressors and oncogenes or otherwise functionally important molecules (e.g. p16, COX, p53, Ki67, etc). In this case, the clinical experts may decide to act upon this new information. Thus, the system may be used in a discovery mode with the expectation that clinical experts will utilize the generated information according to their clinical knowledge of the latest tumor biology.

Optionally, a set of one or more contextualization algorithms **150** implement various methods that use information from a selected one or more of the data modalities **130,** optionally in conjunction with auxiliary disease knowledge **140** from the EHR **20,** PACS **108,** or elsewhere, to perform contextualization which can range from homology assessment to classification of differentially regulated genes (based on RNA, methylation, copy number, or so forth). The computer-implemented algorithms **150** optionally include multiple machine learning tools which then feed their output into tools for visual representation of the data and for interpretation using pathway information.

By way of illustrative example, some examples of interpretation and contextualization that can aid in decision making are described. In a first step after getting the variants, a quality filter is optionally applied. The filter may, for example, include a base calling score, a Phred-like score for single nucleotide polymorphism (SNP) quality, sequencing depth and other proprietary quality measures, or various combinations thereof. A disease association filter is then suitably applied. In a suitable approach, a prioritization step is employed to identify variants that are already reported in an expert-curated disease association database such as dbSNP. Such disease association databases typically focus primarily on nonsynonymous SNP variants since the amino acid change has higher likelihood of impacting the function of the related protein product. However, it is also contemplated to search for other types of SNP variants that can affect function or regulation. For example, it is expected that SNP variants may also be causal for example through influencing promoter activity, the conformation and altered stability of premRNAs which confers through a diminished affinity for a cytoplasmic RNA binding protein, or changing the timing of cotranslational folding. A decision rule is suitably applied to prioritize the matching of these variants, for example by computing a likelihood that the SNP impacts function based on available experimental evidence. Novel but ultraconserved variants can also be prioritized. For the intergenic variants, SNPs that are occurring in the ultraconserved regions are preferably matched first, especially if they belong to a class of noncoding RNAs or a class of repeats that have been reported to associate with disease or functional change.

If the single nucleotide variant is completely novel, further classification of the variant is optionally performed in order to aid the interpretation of single nucleotide variants. This classification is suitably based on dynamically pulling the latest information from the specialized genomic annotation databases (such as the National Center for Bioinformatics, or NCBI) as well as commercial databases if available. For example, transcription factors binding sites database may be available via an institutional contract). Relevant information from these databases may include information as to nonsynonymous ns-SNPs, synonymous sSNPs, and SNPs in the 5'untranslated region (UTR), 3'-UTR, probability of changing splicing sites, as well as intergenic regions. Other relevant information may include databases with continuously updated knowledge about aspects that affect gene regulation such as transcription factors binding site. Another class of relevant information is alternative splicing sites, which can be automatically updated and matched with the variants that may be intersecting these alternative splicing events. These interpretations and exact nature of the class and type of matching are offered to the user to aid in the decision making process.

Novel SNPs that have passed through the foregoing filters can be overlaid on top of graphical models that represent gene interactions. These are either experimentally derived biological pathways or computationally derived gene networks from single or multiple molecular modalities. These biological and computational models are suitably stored in a database in a common format (for example, KGML KEGG markup language or in a more generic XML format) and automatically updated. Associated information about the molecular function (e.g. kinase activity, transcription regulation activity) and/or biological process (e.g. metabolic, chromatin assembly, cell cycle) is also suitably displayed, along with information about location and proximity to regulatory elements which is also displayed in the graphical model.

Decision Support Analytical Applications **170** operate on contextualizers output by the contextualization algorithms **150,** optionally in conjunction with patient clinical data **160,** to generate clinical recommendations. Clinical decision support (CDS) for a given type of clinical application or disease area (e.g. breast cancer) collects data from various information systems in the hospital as well as epidemiological data and intelligently presents the relevant clinical information for each patient case. Optionally, the CDS analytical applications **170** include analytical tools for comparison of patient cases within a patient population. Such CDS applications **170** may employ licensed in-vitro diagnostic tests, e.g. licensed algorithms developed based on clinical studies and relying on certain level of clinical evidence to be recommended for use in the CDS system. Again, the license brokering subsystem **40** ensures compensation due under the license is paid to the licensor, while enabling the clinical staff to perform their diagnostic and decision making tasks without being encumbered with royalty payment concerns.

The illustrative examples set forth herein relate to medical diagnostic tracking, and include the recording of medical diagnostic events related to human subjects including at least the performing of in-vitro diagnostic tests. However, the disclosed approaches are applicable to diagnostic tracking generally, for example in a veterinary context in which the diagnostic tracking includes the recording of diagnostic events related to animal subjects including at least the performing of in-vitro diagnostic tests. As another example, the disclosed approaches are applicable to diagnostic tracking in a lifestyle enhancement or wellness context, in which (by way of illustrative example) the diagnostic tracking may include the recording of diagnostic events including at least the performing of in-vitro diagnostic tests. In a lifestyle enhancement or wellness context, the diagnostic events or tests are directed toward assessing the health or condition of the subject, identifying genetic predisposition toward certain medical conditions such as certain type(s) of cancer (for example, by screening for a biomarker that indicates an enhanced predisposition to breast cancer), or so forth.

This application has described one or more preferred embodiments. Modifications and alterations may occur to others upon reading and understanding the preceding detailed description. It is intended that the application be construed as including all such modifications and alterations insofar as they come within the scope of the appended claims.

**Claims**

1. A method comprising:

     acquiring an extended genomic dataset, which dataset includes data indicative of or relating to an extended portion of a genome;
     processing the extended genomic dataset in silico to discover at least one clinically significant feature of the dataset wherein the processing comprises at least one of (1) performing a biomarker test using the acquired extended genomic dataset, the biomarker test being indicated in a clinical guideline or decision tree and (2) identifying in the extended genomic dataset nucleotide variant information retrieved from one or more genomic databases; and
     generating a clinical result for use in aiding clinical decision making, based on the discovered at least one clinically significant feature of the dataset;
     wherein the processing and generating are performed by one or more computers;
     wherein the processing comprises performing a subset of biomarker tests of a clinical guideline or decision tree with later tests of the subset being chosen dynamically based on results of previously performed biomarker tests in accordance with paths of the clinical guideline or decision tree; and
     wherein the clinical result is generated based on the performed subset of biomarker tests of the clinical guideline or decision tree.

2. The method as set forth in claim **1,** wherein the processing comprises:

     performing one or more first biomarker tests of a clinical guideline or decision tree in silico on the acquired extended genomic dataset; and
     performing the following iteration at least once:

         selecting one or more next biomarker tests of the clinical guideline or decision tree based on biomarker tests of the clinical guideline or decision tree previously performed in silico on the acquired extended genomic dataset, and
         performing the one or more next biomarker tests of the clinical guideline or decision tree in silico on the acquired extended genomic dataset;

     wherein the clinical result is generated based on the performed biomarker tests.

3. The method as set forth in claim **1,** wherein the processing comprises:
filtering the extended genomic dataset in silico to discover at least one clinically significant feature of the dataset wherein the filtering uses nucleotide variant information retrieved from one or more genomic databases wherein the retrieved nucleotide variant information includes information about the nucleotide variant respective to at least impact on function.

4. The method as set forth in claim **1,** wherein the processing comprises:
filtering the extended genomic dataset in silico to discover at least one clinically significant feature of the dataset wherein the filtering uses nucleotide variant information retrieved from one or more genomic databases wherein the retrieved nucleotide variant information includes information about the nucleotide variant respective to at least ultraconserved, promoter activity and alternative splicing impact.

5. The method as set forth in claim **1,** wherein the processing comprises:
filtering the extended genomic dataset in silico to discover at least one clinically significant feature of the dataset wherein the filtering uses nucleotide variant information retrieved from one or more genomic databases wherein the retrieved nucleotide variant information includes information about the nucleotide variant respective to at least single nucleotide polymorphism (SNP) quality.

6. The method as set forth in claim **1,** wherein the processing comprises:
filtering the extended genomic dataset in silico to discover at least one clinically significant feature of the dataset wherein the filtering uses nucleotide variant information retrieved from one or more genomic databases wherein the retrieved nucleotide variant information includes filtering respective to at least (i) single nucleotide polymorphism (SNP) quality followed by (ii) impact on function followed by (iii) ultraconserved, promoter activity and alternative splicing impact.

7. The method as set forth in claim **3,** wherein the processing further comprises:
cross-matching the information retrieved from the one or more genomic databases with novel markers identified in the extended genomic dataset to discover the at least one clinically significant feature.

8. The method as set forth in claim **1,** wherein the acquiring comprises:
acquiring an extended genomic dataset comprising genomic sequencing data using a sequencer.

9. The method as set forth in claim **1,** wherein the acquiring comprises:
acquiring an extended genomic dataset using at least one instrument selected from a group consisting of: a sequencer, a microarray, and a mass spectrometer.

10. The method as set forth in claim **1,** wherein the processing includes performing a biomarker test and the method further comprises:

determining whether the performed biomarker test is a licensed biomarker test based on information stored in a licenses database; and
if the performed biomarker test is a licensed biomarker test then computing license compensation due to a licensor for the performing of the licensed biomarker test based on royalty calculation information stored in the licenses database;
wherein the determining and computing are performed by one or more computers.

11. A storage medium storing instructions executable by a digital processor to perform a method as set forth in claim **1.**

**Patentansprüche**

1. Verfahren, umfassend:

Erfassen eines erweiterten Genomdatensatzes, wobei der Datensatz Daten beinhaltet, die für einen erweiterten Abschnitt eines Genoms indikativ sind oder sich darauf beziehen;
Verarbeiten des erweiterten Genomdatensatzes *in silico,* um mindestens ein klinisch signifikantes Merkmal des Datensatzes zu entdecken, wobei das Verarbeiten mindestens eines umfasst von (1) Durchführen eines Biomarkertests unter Verwendung des erfassten erweiterten Genomdatensatzes, wobei der Biomarkertest in einer klinischen Richtlinie oder einem Entscheidungsbaum angezeigt ist, und (2) Identifizieren in dem erweiterten Genomdatensatz von Nukleotidvarianteninformationen, die aus einer oder mehreren Genomdatenbanken abgerufen werden; und
Erzeugen eines klinischen Resultats zur Verwendung bei der Unterstützung klinischer Entscheidungsfindung basierend auf dem entdeckten mindestens einem klinisch signifikanten Merkmal des Datensatzes;
wobei das Verarbeiten und Erzeugen von einem oder mehreren Computern durchgeführt wird;
wobei das Verarbeiten Durchführen eines Untersatzes von Biomarkertests einer klinischen Richtlinie oder eines Entscheidungsbaums umfasst, wobei später Tests des Untersatzes dynamisch basierend auf Resultaten der vorher durchgeführten Biomarkertests gemäß Pfaden der klinischen Richtlinie oder des Entscheidungsbaums durchgeführt werden; und
wobei das klinische Resultat basierend auf dem durchgeführten Untersatz der Biomarkertests der klinischen Richtlinie oder des Entscheidungsbaums erzeugt wird.

2. Verfahren, wie in Anspruch 1 dargelegt, wobei das Verarbeiten umfasst:

Durchführen von einem oder mehreren ersten Biomarkertests einer klinischen Richtlinie oder eines Entscheidungsbaums *in silico* an dem erfassten erweiterten Genomdatensatz; und Durchführen der folgenden Iteration mindestens einmal:

Auswählen von einem oder mehreren nächsten Biomarkertests der klinischen Richtlinie oder des Entscheidungsbaums basierend auf Biomarkertests der klinischen Richtlinie oder des Entscheidungsbaums, die zuvor *in silico* an dem erfassten erweiterten Genomdatensatz durchgeführt wurden, und

Durchführen des einen oder der mehreren nächsten Biomarkertests der klinischen Richtlinie oder des Entscheidungsbaums *in silico* an dem erfassten erweiterten Genomdatensatz;

wobei das klinische Resultat basierend auf den durchgeführten Biomarkertests erzeugt wird.

3. Verfahren, wie in Anspruch 1 dargelegt, wobei das Verarbeiten umfasst:
Filtern des erweiterten Genomdatensatzes *in silico,* um mindestens ein klinisch signifikantes Merkmal des Datensatzes zu entdecken, wobei das Filtern Nukleotidvarianteninformationen verwendet, die aus einer oder mehreren Genomdatenbanken abgerufen wurden, wobei die abgerufenen Nukleotidvarianteninformationen Informationen über die Nukleotidvariante mit Bezug auf mindestens Auswirkung auf Funktion beinhaltet.

4. Verfahren, wie in Anspruch 1 dargelegt, wobei das Verarbeiten umfasst:
Filtern des erweiterten Genomdatensatzes *in silico,* um mindestens ein klinisch signifikantes Merkmal des Datensatzes zu entdecken, wobei das Filtern Nukleotidvarianteninformationen verwendet, die aus einer oder mehreren Genomdatenbanken abgerufen wurden, wobei die abgerufenen Nukleotidvarianteninformationen Informationen über die Nukleotidvariante mit Bezug auf mindestens ultrakonserviert, Promoteraktivität und alternative Spleißwirkung beinhalten.

5. Verfahren, wie in Anspruch 1 dargelegt, wobei das Verarbeiten umfasst:
Filtern des erweiterten Genomdatensatzes *in silico,* um mindestens ein klinisch signifikantes Merkmal des Datensatzes zu entdecken, wobei das Filtern Nukleotidvarianteninformationen verwendet, die aus einer oder mehreren Genomdatenbanken abgerufen wurden, wobei die abgerufenen Nukleotidvarianteninformationen Informationen über die Nukleotidvariante mit Bezug auf mindestens eine einzelne Nukleotidpolymorphismus (SNP) Qualität beinhalten.

6. Verfahren, wie in Anspruch 1 dargelegt, wobei das Verarbeiten umfasst:
Filtern des erweiterten Genomdatensatzes *in silico,* um mindestens ein klinisch signifikantes Merkmal des Datensatzes zu entdecken, wobei das Filtern Nukleotidvarianteninformationen verwendet, die aus einer oder mehreren Genomdatenbanken abgerufen wurden, wobei die abgerufenen Nukleotidvarianteninformationen Filtern beinhaltet, das sich mindestens bezieht auf (i) einzelne Nukleotidpolymorphismus (SNP) Qualität, gefolgt von (ii) Auswirkung auf Funktion, gefolgt von (iii) ultrakonserviert, Promoteraktivität und alternative Spleißwirkung.

7. Verfahren, wie in Anspruch 3 dargelegt, wobei das Verarbeiten ferner umfasst:
Kreuzabgleichen der Informationen, die aus der einen oder den mehreren Genomdatenbanken abgerufen wurden, mit neuen Markern, die in dem erweiterten Genomdatensatz identifiziert wurden, um das mindestens eine klinisch signifikante Merkmal zu entdecken.

8. Verfahren, wie in Anspruch 1 dargelegt, wobei das Erfassen umfasst:
Erfassen eines erweiterten Genomdatensatzes, der Genomsequenzierungsdaten unter Verwendung eines Sequenzierers.

9. Verfahren, wie in Anspruch 1 dargelegt, wobei das Erfassen umfasst:
Erfassen eines erweiterten Genomdatensatzes unter Verwendung mindestens eines Instruments, das aus einer Gruppe ausgewählt wird, die besteht aus: einem Sequenzierer, einem Microarray und einem Massenspektrometer.

10. Verfahren, wie in Anspruch 1 dargelegt, wobei das Verarbeiten Durchführen eines Biomarkertests beinhaltet und das Verfahren ferner umfasst:

Bestimmen, ob der durchgeführte Biomarkertest ein lizenzierter Biomarkertest basierend auf Informationen ist, die in einer Lizenzdatenbank gespeichert sind; und
falls der durchgeführte Biomarkertest ein lizenzierter Biomarkertest ist, dann berechnen einer an einen Lizenzgeber fälligen Lizenzkompensation für das Durchführen des lizenzierten Biomarkertests basierend auf Lizenzgebührenberechnungsinformationen, die in der Lizenzdatenbank gespeichert sind;
wobei das Bestimmen und Berechnen von einem oder mehreren Computern durchgeführt wird.

11. Speichermedium, das Anweisungen speichert, die von einem digitalen Prozessor zum Durchführen eines Verfahrens, wie in Anspruch 1 dargelegt, ausführbar sind.

**Revendications**

1. Procédé comprenant :

l'acquisition d'un ensemble de données génomiques étendu, lequel ensemble de données inclut des données indiquant ou concernant une partie étendue d'un génome ;

le traitement de l'ensemble de données génomiques étendu in silico pour découvrir au moins une caractéristique cliniquement significative de l'ensemble de données dans lequel le traitement comprend au moins soit (1) la réalisation d'un test de marqueur biologique à l'aide de l'ensemble de données génomiques étendu acquis, le test de marqueur biologique étant indiqué dans une directive clinique ou un arbre de décision, soit (2) l'identification dans l'ensemble de données génomiques étendu d'informations sur un variant nucléotidique extraites d'une ou plusieurs bases de données génomiques ; et

la production d'un résultat clinique destiné à faciliter la prise de décision clinique, sur la base de l'au moins une caractéristique cliniquement significative découverte de l'ensemble de données ;

dans lequel le traitement et la production sont réalisés par un ou plusieurs ordinateurs ;

dans lequel le traitement comprend la réalisation d'un sous-ensemble de tests de marqueurs biologiques d'une directive clinique ou d'un arbre de décision, des tests ultérieurs du sous-ensemble étant choisis dynamiquement sur la base de résultats de tests de marqueurs biologiques précédemment réalisés conformément à des chemins de la directive clinique ou de l'arbre de décision ; et

dans lequel le résultat clinique est produit sur la base du sous-ensemble réalisé de tests de marqueurs biologiques de la directive clinique ou de l'arbre de décision.

2. Procédé selon la revendication 1, dans lequel le traitement comprend :

la réalisation d'un ou plusieurs premiers tests de marqueurs biologiques d'une directive clinique ou d'un arbre de décision in silico sur l'ensemble de données génomiques étendu acquis ; et

la réalisation de l'itération suivante au moins une fois :

sélection d'un ou plusieurs tests de marqueurs biologiques suivants de la directive clinique ou de l'arbre de décision sur la base de tests de marqueurs biologiques de la directive clinique ou de l'arbre de décision précédemment réalisés in silico sur l'ensemble de données génomiques étendu acquis, et

réalisation des un ou plusieurs tests de marqueurs biologiques suivants de la directive clinique ou de l'arbre de décision in silico sur l'ensemble de données génomiques étendu acquis ;

dans lequel le résultat clinique est produit sur la base des tests de marqueurs biologiques réalisés.

3. Procédé selon la revendication 1, dans lequel le traitement comprend :
le filtrage de l'ensemble de données génomiques étendu in silico pour découvrir au moins une caractéristique cliniquement significative de l'ensemble de données dans lequel le filtrage utilise des informations sur un variant nucléotidique extraites d'une ou plusieurs bases de données génomiques dans lequel les informations sur le variant nucléotidique extraites incluent des informations sur le variant nucléotidique relatives au moins à son impact sur une fonction.

4. Procédé selon la revendication 1, dans lequel le traitement comprend :
le filtrage de l'ensemble de données génomiques étendu in silico pour découvrir au moins une caractéristique cliniquement significative de l'ensemble de données dans lequel le filtrage utilise des informations sur un variant nucléotidique extraites d'une ou plusieurs bases de données génomiques dans lequel les informations sur le variant nucléotidique extraites incluent des informations sur le variant nucléotidique relatives au moins à son activité promotrice ultraconservée et à son impact sur l'épissage alternatif.

5. Procédé selon la revendication 1, dans lequel le traitement comprend :
le filtrage de l'ensemble de données génomiques étendu in silico pour découvrir au moins une caractéristique cliniquement significative de l'ensemble de données dans lequel le filtrage utilise des informations sur un variant nucléotidique extraites d'une ou plusieurs bases de données génomiques dans lequel les informations sur le variant nucléotidique extraites incluent des informations sur le variant nucléotidique relatives au moins à la qualité du polymorphisme mononucléotidique (SNP).

6. Procédé selon la revendication 1, dans lequel le traitement comprend :

le filtrage de l'ensemble de données génomiques étendu in silico pour découvrir au moins une caractéristique cliniquement significative de l'ensemble de données dans lequel le filtrage utilise des informations sur un variant nucléotidique extraites d'une ou plusieurs bases de données génomiques dans lequel les informations sur le variant nucléotidique extraites incluent le filtrage relatif au moins à (i) la qualité du polymorphisme mononucléotidique (SNP) suivie de (ii) l'impact sur une fonction suivi de (iii) l'activité promotrice ultraconservée et l'impact sur l'épissage alternatif.

7. Procédé selon la revendication 3, dans lequel le traitement comprend en outre :
le recoupement des informations extraites des une ou plusieurs bases de données génomiques avec de nouveaux marqueurs identifiés dans l'ensemble de données génomiques étendu pour découvrir l'au moins une caractéristique cliniquement significative.

8. Procédé selon la revendication 1, dans lequel l'acquisition comprend :
l'acquisition d'un ensemble de données génomiques étendu comprenant des données de séquençage génomique à l'aide d'un séquenceur.

9. Procédé selon la revendication 1, dans lequel l'acquisition comprend :
l'acquisition d'un ensemble de données génomiques étendu à l'aide d'au moins un instrument choisi dans un groupe consistant en : un séquenceur, un microréseau, et un spectromètre de masse.

10. Procédé selon la revendication 1, dans lequel le traitement inclut la réalisation d'un test de marqueur biologique et le procédé comprend en outre :

le fait de déterminer si le test de marqueur biologique réalisé est, ou non, un test de marqueur biologique sous licence sur la base d'informations stockées dans une base de données de licences ; et
si le test de marqueur biologique réalisé est un test de marqueur biologique sous licence, alors le calcul d'une contrepartie de licence due à un concédant de licence pour la réalisation du test de marqueur biologique sous licence sur la base d'informations sur le calcul de royalties stockées dans la base de données de licences ;
dans lequel la détermination et le calcul sont réalisés par un ou plusieurs ordinateurs.

11. Support de stockage stockant des instructions exécutables par un processeur numérique pour mettre en oeuvre un procédé selon la revendication 1.

FIG. 1

```
┌─────────────────────────────────────┐
│        Select applicable tests       │──── R1
└─────────────────────────────────────┘
                    │
                    ▼
┌─────────────────────────────────────┐
│      Prioritize based on levels of   │
│     guidelines and FDA approval:     │──── R2
│      assign weights {W1,...,Wn}      │
└─────────────────────────────────────┘
                    │
                    ▼
┌─────────────────────────────────────┐
│     Prioritize based on impact to    │
│          medical condition:          │──── R3
│       assign weights {V1,...,Vk}     │
└─────────────────────────────────────┘
                    │
                    ▼
┌─────────────────────────────────────┐
│       Select m tests of n available  │──── R4
└─────────────────────────────────────┘
                    │
                    ▼
┌─────────────────────────────────────┐
│       Lookup Total Insurance         │
│          recommended                 │──── R5
│          reimbursement               │
└─────────────────────────────────────┘
                    │
                    ▼
┌─────────────────────────────────────┐
│        Reimbursement_i =             │
│      (Total_reimbursement /          │──── R6
│         m+p)*(Vi+Wi)                 │
└─────────────────────────────────────┘
```

# FIG. 2

```
┌─────────────────────────────────────────────┐
│          Initial breast cancer diagnosis     │──── S1
└─────────────────────────────────────────────┘
                      │
                      ▼
┌─────────────────────────────────────────────┐
│        Patient EHR created or updated to      │──── S2
│       include tentative breast cancer diagnosis│
└─────────────────────────────────────────────┘
                      │
                      ▼
┌─────────────────────────────────────────────┐
│         Patient record created on CDS components│
│       and linked with breast cancer clinical guideline│──── S3
│          and guideline is displayed to physician│
└─────────────────────────────────────────────┘
                      │
                      ▼
┌─────────────────────────────────────────────┐
│       Physician decides to order guideline assays│──── S4
└─────────────────────────────────────────────┘
                      │
                      ▼
┌─────────────────────────────────────────────┐
│      CDS components identify guideline assays and│
│       other assays that can be performed with the│
│       sequencing data generated for the guideline│──── S5
│        assays, invokes license brokering sub-module│
│           to identify any proprietary test issues│
└─────────────────────────────────────────────┘
                      │
                      ▼
┌─────────────────────────────────────────────┐
│          Physician selects assays to perform  │──── S6
└─────────────────────────────────────────────┘
                      │
                      ▼
┌─────────────────────────────────────────────┐
│      Perform sequencing of tumor DNA, germline,│
│     normal adjacent tissue, and metastases as required│──── S7
│           by the assays ordered by the physician│
└─────────────────────────────────────────────┘
                      │
                      ▼
┌─────────────────────────────────────────────┐
│     Perform assembly and alignment against reference│──── S8
└─────────────────────────────────────────────┘
                      │
                      ▼
┌─────────────────────────────────────────────┐
│     Aligned and optionally enriched sequencing data│──── S10
└─────────────────────────────────────────────┘
```

FIG. 3

| Aligned and optionally enriched sequencing data | ~ S10 |

↓

| Perform assays ordered by physician (mutations, CNVs, methylation, expression, etc) | ~ S11 |

↓

| Record assays and invoke license brokering sub-module to resolve any royalty reimbursements | ~ S12 |

↓

| Physician reviews results and makes clinical decision (treatment, prophylaxis, ...) | ~ S13 |

↓

| Physician decides to request an additional assay employing the generated sequencing profile | ~ S14 |

## FIG. 4

Guideline assays include sequencing of the following: ////////

The following assays can be performed using this sequencing data:

Guideline assays: ■ Breast assay "X"
■ Breast assay "Y"          See note A

Additional available assays:

■ Colon assay  "N"
☐ Rectal assay  "V"          See note B
☐ Breast assay  "Y_S"          See note C

Note A:  Breast assay "Y" requires a royalty of $78.00 payable upon performing the assay. This royalty is not covered by Acme Insurance Company.

Note B:  This assay is proprietary, and can only be performed by NeuavoLobo Labs.

Note C:  Breast assay "Y_S" is accredited by the British National Institute for Clinical Excellence (NICE) as equivalent to Breast Assay "Y", but is not accredited by any accreditation agency in the United States.

Please check the assays to be performed, and press (CONTINUE)

FIG. 5

EP 2 636 003 B1

```
┌─────────────────────────────────────────────┐
│            Select decision tree             │──── E1
└─────────────────────────────────────────────┘
                      │
                      ▼
┌─────────────────────────────────────────────┐
│       Identify extended genomic dataset      │──── E2
└─────────────────────────────────────────────┘
                      │
                      ▼
┌─────────────────────────────────────────────┐
│  Apply license brokering sub-system to exclude │
│  any extraneous licensed data from the dataset │──── E3
└─────────────────────────────────────────────┘
                      │
                      ▼
┌─────────────────────────────────────────────┐
│  Targeted acquisition of extended genomic dataset │──── E4
└─────────────────────────────────────────────┘
                      │
                      ▼
┌─────────────────────────────────────────────┐
│         Record acquisition and apply license    │
│          brokering sub-system to pay any        │──── E5
│        royalties associated with the acquisition │
└─────────────────────────────────────────────┘
                      │
                      ▼
┌─────────────────────────────────────────────┐
│           Perform test(s) identified by        │
│            the decision tree in silico         │──── E6
└─────────────────────────────────────────────┘
                      │
                      ▼
┌─────────────────────────────────────────────┐
│      Record test(s) and apply license brokering  │
│     sub-system to pay any royalties associated   │──── E7
│            with performing the tests            │
└─────────────────────────────────────────────┘
                      │
                      ▼
┌─────────────────────────────────────────────┐
│          Identify next test(s) based           │
│         on results and decision tree           │──── E8
└─────────────────────────────────────────────┘
                      │
                      ▼
                  ╱─────────╲
                 ╱  Repeat    ╲
                ╱ until decision ╲──── E9
                ╲ tree terminates ╱
                 ╲─────────────╱
```

## FIG. 6

FIG. 7

FIG. 8

130

Modalities

Gene
expression

DNA
Methylation

SNP/CNP/
Mutations

TF binding

Regulatory
(miRNA)

Protein
expression

Data conduits

150

Contextuallization
algorithms
Disease specific

Pathway
assessmenty

Visualization/
contextualization

Classification

Homology
assessment

In-silico
design

Contextualizers

170

Clinical
Decision support

Early Dx and
subtyping

Patient
stratification

Therapy Planning
& Outcome
Prediction

Therapy
Monitoring

Evidence based
medicine

Auxiliary
disease
knowledge

140

Patient
clinical
data

160

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004044236 A
- WO 2008067551 A

- WO 0126029 A